# EUROPEAN PATENT APPLICATION

(11) **EP 3 690 034 A1**
(43) Date of publication of application: **05.08.2020**
(21) Application number: 18861531.4
(22) Date of filing: 28.06.2018
(51) Int. Cl.: C12N 7/01, C12N 15/09, A61K 35/768, A61K 39/285, A61K 48/00, A61P 35/00

(54) **ISOLATED RECOMBINANT ONCOLYTIC POXVIRUS, PHARMACEUTICAL COMPOSITION, AND USE THEREOF IN TREATMENT OF TUMORS AND/OR CANCER**

(30) Priority: 26.09.2017 CN 201710882027
(71) Applicant: Hangzhou Converd Co., Ltd., Hangzhou, Zhejiang 311121 (CN)
(72) Inventor: FU, Jin, Hangzhou Zhejiang 311121 (CN); ZHAO, Ronghua, Hangzhou Zhejiang 311121 (CN); ZHANG, Rong, Hangzhou Zhejiang 311121 (CN); WANG, Tingting, Hangzhou Zhejiang 311121 (CN); CHEN, Lin, Hangzhou Zhejiang 311121 (CN); HU, Fang, Hangzhou Zhejiang 311121 (CN)
(74) Representative: Cohausz & Florack
(86) International application number: PCT/CN2018/093404
(87) International publication number: WO 2019/062234

(57) **Abstract**

The invention provides an isolated recombinant oncolytic vaccinia virus, pharmaceutical compositions and uses thereof for drugs for treatment of tumors and/or cancers. The isolated recombinant oncolytic vaccinia virus is functionally deficient in the TK gene and the VGF gene, and the genome of the recombinant oncolytic vaccinia virus is integrated with an exogenous IL-21 gene, and wherein the IL-21 gene is capable of being expressed in tumor cells. The recombinant oncolytic vaccinia virus can selectively replicate in tumor cells, and can also fully exert the anti-tumor immune effect of the exogenous IL-21, such that the oncolytic killing effect of the oncolytic virus and the anti-tumor immune stimulation effect of IL-21 achieve a synergic effect.

SEQUENCE LISTING

<110>  Hangzhou ConVerd Co., Ltd.

<120>  Isolated Recombinant Oncolytic Vaccinia Viruses, Pharmaceutical
Compositions and Uses Thereof for Drugs for Treatment of Tumors and/or
Cancers

<130>  UP-182214-59:52

<150>  CN201710882027.1

<151>  2017-09-26

<160>  17

<170>  PatentIn version 3.5

<210>  1
<211>  501
<212>  DNA
<213>  Mus musculus
<400>  1
agatctatgg agaggaccct tgtctgtctg gtagtcatct tcttggggac agtggcccat      60

aaatcaagcc cccaagggcc agatcgcctc ctgattagac ttcgtcacct tattgacatt     120

gttgaacagc tgaaaatcta tgaaaatgac ttggatcctg aacttctatc agctccacaa     180

gatgtaaagg ggcactgtga gcatgcagct tttgcctgtt ttcagaaggc caaactcaag     240

ccatcaaacc ctggaaacaa taagacattc atcattgacc tcgtggccca gctcaggagg     300

aggctgcctg ccaggagggg aggaaagaaa cagaagcaca tagctaaatg cccttcctgt     360

gattcgtatg agaaaaggac acccaaagaa ttcctagaaa gactaaaatg gctccttcaa     420

aaggtatgca ccttaaatgc atttctttca cttccatgtt gtgtccgggt acctcctgtg     480

cccagtgact catagactag t                                               501


<210>  2
<211>  501
<212>  DNA
<213>  Homo sapiens

<400>  2
agatctatga gatccagtcc tggcaacatg gagaggattg tcatctgtct gatggtcatc      60

ttcttgggga cactggtcca caaatcaagc tcccaaggtc aagatcgcca catgattaga     120

atgcgtcaac ttatagatat tgttgatcag ctgaaaaatt atgtgaatga cttggtccct     180

gaatttctgc cagctccaga agatgtagag acaaactgtg agtggtcagc tttttcctgc     240

tttcagaagg cccaactaaa gtcagcaaat acaggaaaca atgaaaggat aatcaatgta     300

tcaattaaaa agctgaagag gaaaccacct tccacaaatg cagggagaag acagaaacac     360

agactaacat gcccttcatg tgattcttat gagaaaaaac cacccaaaga attcctagaa     420

agattcaaat cacttctcca aaagatgatt catcagcatc tgtcctctag aacacacgga     480

agtgaagatt cctgaactag t 501

<210> 3
<211> 24
<212> DNA
<213> Artificial Sequence

<400> 3
atcgcatttt ctaacgtgat ggat 24

<210> 4
<211> 23
<212> DNA
<213> Artificial Sequence

<400> 4
tatctaacga cacaacatcc att 23

<210> 5
<211> 21
<212> DNA
<213> Artificial Sequence

<400> 5
atggaatccg cgtcgactga g 21

<210> 6
<211> 22
<212> DNA
<213> Artificial Sequence

<400> 6
ggccagatcg cctcctgatt ag 22

<210> 7
<211> 21
<212> DNA
<213> Artificial Sequence

<400> 7
ggtccctgaa tttctgccag c 21

<210> 8
<211> 18
<212> DNA
<213> Artificial Sequence

<400> 8
cagggaaacg gatgtata 18

<210> 9
<211> 22
<212> DNA
<213> Artificial Sequence

<400>  9
tgtgttacag aatcatataa gg                                                22

<210>  10
<211>  24
<212>  DNA
<213> Artificial Sequence

<400>  10
ccaactaaag tcagcaaata cagg                                              24

<210>  11
<211>  23
<212>  DNA
<213> Artificial Sequence

<400>  11
ctttctagga attctttggg tgg                                               23

<210>  12
<211>  26
<212>  DNA
<213> Artificial Sequence

<400>  12
ggcttttatc cacaagaatc aagatc                                            26

<210>  13
<211>  20
<212>  DNA
<213> Artificial Sequence

<400>  13
gtgcacgtct accgcagaga                                                   20

<210>  14
<211>  22
<212>  DNA
<213> Artificial Sequence

<400>  14
agtgtggaga ccatcaagga ag                                                22

<210>  15
<211>  21
<212>  DNA
<213> Artificial Sequence

<400>  15
gtattgcttt gcgttggaca t                                                 21

<210>  16
<211>  20
<212>  DNA
<213> Artificial Sequence

<400>  16
ggtctcctct gacttcaaca                                                    20

<210>  17
<211>  20
<212>  DNA
<213> Artificial Sequence

<400>  17
agccaaattc gttgtcatac                                                    20

## Description

### TECHNICAL FIELD

The instant invention belongs to the field of biotechnology and, specifically, relates to isolated recombinant oncolytic vaccinia viruses, pharmaceutical compositions and uses thereof for drugs for treatment of tumors and/or cancers.

### BACKGROUND

An oncolytic virus is a type of virus that selectively replicates in tumor cells and kills tumor cells, but does not harm normal cells. After replicating in the infected tumor cells, new virus particles released, which in turn infects the tumor cells around them, and further exert an oncolytic effect. In addition to this direct oncolytic effect, the oncolytic viruses can effectively stimulate the body to produce an immune response against the virus itself and against the infected tumor cells. It can be seen that the anti-tumor effect of the oncolytic viruses is achieved by both selectively killing tumor cells and inducing the body to produce a systematic anti-tumor immune response.

As early as the end of the 19th century, multiple viruses were found to alleviate the progression of tumor development, indicating the potential of the virus in the field of tumor therapy. With the development of genetic technology, the virus genome structure can be modified so that it can be selectively replicated within tumor cells, thereby enhancing its tumor-targeted property. In the past ten years, the researchers have developed a series of oncolytic virus products by genetically recombination, gene transfer, gene knockout and other technologies to genetically modify adenoviruses, herpes virus, small RNA virus, vaccinia virus and other viruses. To date, more than 20 products have entered into different stages of clinical research. Among them, China CFDA approved the marketing of the gene-modified oncolytic adenoviruses H101 of Shanghai Sunway Biotech Co., Ltd. for the treatment of head and neck tumors in 2005, which was the world's first oncolytic virus drug. Ten years later, a second oncolytic virus drug, Amgen's gene-modified oncolytic herpes virus, T-Vec, was approved by US FDA and European Union EMA for the treatment of advanced malignant melanoma in 2015. There is currently no genetically modified oncolytic vaccinia virus on the market as a drug.

There is still a need to develop more effective medicine in the immunotherapy of tumor and/or cancer by oncolytic viruses.

### SUMMARY

In order to solve the existing problems in the prior art, the present invention provides an isolated recombinant oncolytic vaccinia virus, pharmaceutical compositions and uses thereof for drugs for treatment of tumors and/or cancers.

Specifically, the present invention provides:
(1) An isolated recombinant oncolytic vaccinia virus, wherein the recombinant oncolytic vaccinia virus is functionally deficient in the TK gene and the VGF gene, and the genome of the recombinant oncolytic vaccinia virus is integrated with an exogenous IL-21 gene, and wherein the IL-21 gene is capable of being expressed in tumor cells.
(2) The recombinant oncolytic vaccinia virus according to (1), wherein the TK gene gets functional deficient by being inserted an exogenous nucleotide sequence.
(3) The recombinant oncolytic vaccinia virus according to (1), wherein the exogenous IL-21 gene is inserted into the TK gene, thereby causing functional defect of the TK gene.
(4) The recombinant oncolytic vaccinia virus according to (1), wherein the VGF gene gets functional deficient by being knocked out or being inserted an exogenous nucleotide sequence.
(5) The recombinant oncolytic vaccinia virus according to (1), wherein the recombinant oncolytic vaccinia virus is a Wyeth strain or WR strain.
(6) The recombinant oncolytic vaccinia virus according to (1), wherein the genome of the recombinant oncolytic vaccinia virus is further integrated with an exogenous screening gene and the exogenous screening gene includes the gpt gene and/or LacZ gene, but do not include fluorescent protein genes.
(7) The recombinant oncolytic vaccinia virus according to (1) or (5), wherein the exogenous IL-21 gene is derived from mouse or human.
(8) A pharmaceutical composition wherein the pharmaceutical composition comprises the recombinant oncolytic vaccinia virus according to any one of (1)-(7) as an active ingredient, and a pharmaceutically acceptable excipient.
(9) The pharmaceutical composition according to (8), wherein the pharmaceutical composition comprises the recombinant oncolytic vaccinia virus at a dose of 1 × 10⁵-5 × 10⁹ pfu/day.
10) The pharmaceutical composition according to (8), wherein the recombinant oncolytic vaccinia virus is administered by intratumoral injection or intravenous administration.
(11) A vector for preparing the recombinant oncolytic vaccinia virus according to any one of (1) to (7), wherein the vector comprises an exogenous IL-21 gene under the control of a promoter.
(12) A host cell comprising the vector according to (11).
(13) Use of the recombinant oncolytic vaccinia virus according to any one of (1) to (7) for preparation of drugs for treatment of tumors and/or cancers.
(14) The use according to (13), wherein the tumors and/or cancers include lung cancer, melanoma, head and neck cancer, liver cancer, brain cancer, colorectal cancer, bladder cancer, breast cancer, ovarian cancer, uterus cancer, cervical cancer, lymphoma, stomach cancer, esophageal cancer, kidney cancer, prostate cancer, pancreatic cancer, leukemia, bone cancer, testicular cancer.
(15) A method for treating tumors and/or cancers, comprising administering the recombinant oncolytic vaccinia virus according to any one of (1) to (7) to a tumor and /or cancer patient.
(16) The method according to (15), wherein the recombinant oncolytic vaccinia virus is administered at a dose of 1 × 10⁵-5 × 10⁹ pfu/day, once a day for 1-6 consecutive days.
(17) The method according to (15), wherein the recombinant oncolytic vaccinia virus is administered by intratumoral injection or intravenous administration.
(18) The method according to (15), wherein the tumors and /or cancers include lung cancer, melanoma, head and neck cancer, liver cancer, brain cancer, colorectal cancer, bladder cancer, breast cancer, ovarian cancer, uterus Cancer, cervical cancer, lymphoma, stomach cancer, esophageal cancer, kidney cancer, prostate cancer, pancreatic cancer, leukemia, bone cancer, testicular cancer.

Compared with the prior art, the present invention has the following advantages and positive effects:
The invention proposes for the first time that making both of the TK gene and the VGF gene of the an oncolytic vaccinia virus functional deficient, and at the same time making the oncolytic vaccinia virus carry the gene of the immune regulatory factor IL-21, so that the obtained recombinant oncolytic vaccinia virus can selectively replicate in tumor cells, and expresses the immune regulatory factor IL-21. The oncolytic vaccinia virus, the pharmaceutical composition, and the method provided based on the present conception can fully exert the roles of the oncolytic virus to selectively replicate in tumor cells and kill tumor cells, and to further cause the subsequent immune response in the body, and also can fully exert the antitumor immune effect of the exogenous IL-21. The invention finds that by integrating the IL-21 gene in the oncolytic vaccinia virus, the present invention can synergize the oncolytic killing effect of the oncolytic virus and the antitumor immune stimulating effect of IL-21.

In addition, the functional defects in both of the TK and VGF genes of the vaccinia virus effectively enhance its tumor targeting property, thereby improving the safety.

In addition, since the recombinant oncolytic vaccinia virus of the present invention selectively replicates in tumor cells while expressing exogenous IL-21, it can synergistically stimulate the body's anti-tumor immune response, thereby enabling the recombinant oncolytic vaccinia virus of the present invention to combine with NK cells. The pharmaceutical composition and the method provided based on this conception can not only fully play the roles of the recombinant oncolytic vaccinia virus of the present invention to selectively replicate in tumor cells and kill tumor cells, and to further cause the subsequent immune response of the body, but also exert the function of NK cells to kill tumor cells, and skillfully taking advantage of the characteristic of the recombinant oncolytic vaccinia virus of the present invention that selectively replicates in tumor cells, so that the tumor cells containing the recombinant oncolytic vaccinia virus of the present invention become the specific targets of NK cells. The expressed exogenous IL-21 can also enhance the lethality of NK cells, thereby further enhancing the tumor-killing effect of NK cells. This ultimately produces a further enhanced synergistic tumor killing effect.

Further, through research, the respective administration dose, administration sequence, and administration interval of the recombinant oncolytic vaccinia virus and NK cells proposed by the present invention enables the combination thereof to achieve the maximum synergistic effect while avoiding the mutual constraints among them, so as to achieve effective effect of treating tumors and /or cancers.

### Definition

As used herein, the terms "tumor", "cancer", "tumor cell" and "cancer cell" cover the meanings generally recognized in the art.

As used herein, the term "oncolytic virus" refers to a virus that can replicate selectively in and lyse tumor cells.

As used herein, the term "therapeutically effective dose" refers to an amount of a functional agent or of a pharmaceutical composition useful for exhibiting a detectable therapeutic or inhibitory effect or invoking an antitumor response. The effect can be detected by any assay method known in the art.

As used herein, the term "administer" or "administration" refers to providing a compound, a composite or a composition (including viruses and cells) to a subject.

As used herein, the term "patient" refers to a human or non-human organism. Thus, the methods and compositions described herein are applicable to both human and veterinary diseases. In certain embodiments, the patient has a tumor. In some cases, the patient may suffer from one or more types of cancer simultaneously.

As used herein, the term "synergistic effect" refers to an effect arising between two or more agents that produce an effect greater than the sum of their individual effects.

As used herein, the term "pfu", or "plaque forming unit" refers to the number of viruses forming a plaque.

As used herein, the term "MOI", or "multiplicity of infection" refers to the ratio between the number of viruses and the number of cells, i.e., the number of virus particles used to initiate viral infection per cell. MOI = pfu/cell, that is, the number of cells × MOI = Total PFU.

### BRIEF DESCRIPTION OF THE FIGURES

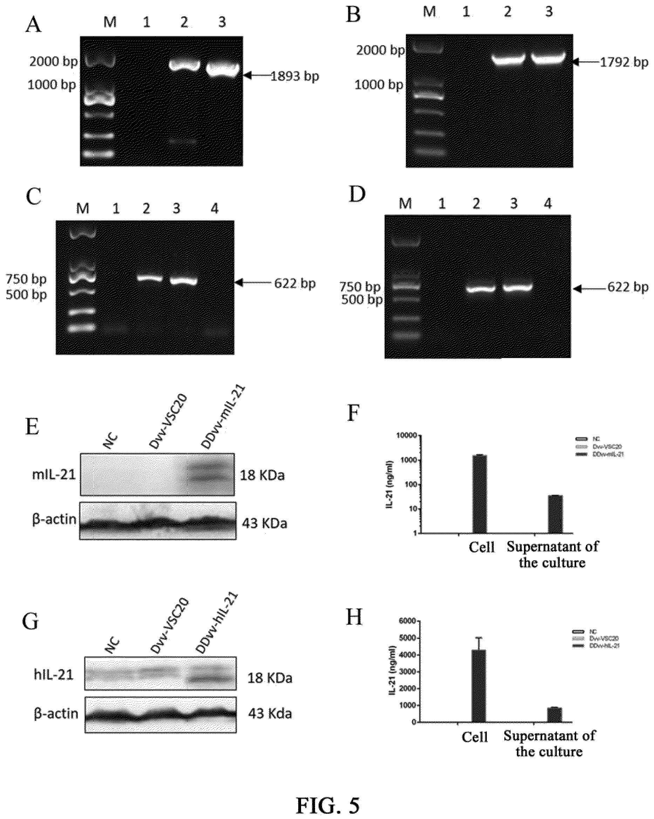

FIG. 1 shows a profile of a recombinant plasmid constructed to insert the IL-21 gene into the TK gene of the vaccinia virus VSC20 (that is, "Dvv-VSC20 / VGF-" shown in the figure) and a schematic diagram of the recombination mechanism according to an embodiment of the present invention, wherein a recombinant double gene-deficient vaccinia virus (recombinant DDvv) according to one embodiment of the present invention is obtained through the shown recombination mechanism.
Figure 2 shows a flowchart of one embodiment for obtaining the recombinant oncolytic vaccinia virus of the present invention.
FIG. 3 shows a profile of the plasmid constructed in Example 1 of the present invention.

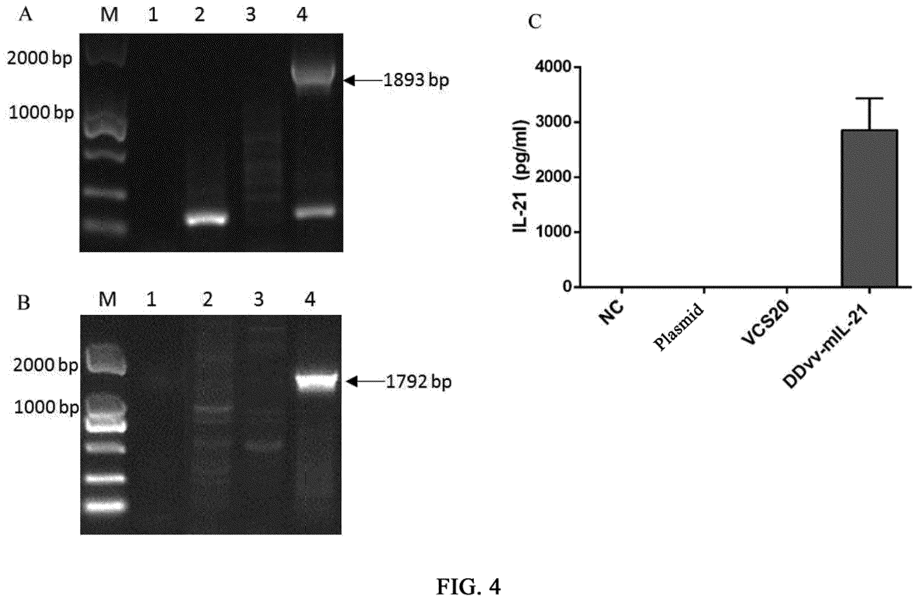

Fig. 4 shows the results of identifying the P0-generation recombinant virus DDvv-IL21 by PCR and ELISA methods; wherein A is detection of the P0-generation virus DDvv-mIL21 by PCR method, and B is detection of the P0-generation virus DDvv-hIL21 by PCR method; wherein, lane M: D000 marker, lane 1: negative control (i.e., PCR reaction solution), lane 2: VSC20, lane 3: CV1 cells, lane 4: P0 generation virus; C shows the expression level of IL21 in the culture supernatant of P0 generation virus identified by ELISA. In Fig. 4C, the abscissa is the groups, and the ordinate is the concentration of IL21 (pg / mL); "NC" shown on the abscissa refers to CV1 cells, and "plasmid" refers to pCB-mIL21.
Figure 5 shows the identification results of the recombinant virus after plaque screening;
where A and B are the identification of oncolytic vaccinia viruses DDvv-mIL21 and
DDvv-hIL21, respectively, by PCR; wherein, lane M: D000 marker, lane 1: negative control (That is, PCR reaction solution), lane 2: P0 generation virus, lane 3: DDvv-mIL21 (Figure 5A) or DDvv-hIL21 (Figure 5B). C and D are the TK region identification by PCR method, wherein lane M in the figure: D000 marker, lane 1: negative control (i.e., PCR reaction solution), lane 2: VSC20, lane 3: P0 generation virus, lane 4: DDvv-mIL21 (Figure 5C) or DDvv-hIL21 (Figure 5D). E and G show the expression of IL21 protein in the oncolytic vaccinia viruses DDvv-mIL21 and DDvv-hIL21 respectively via Western Blot using anti-IL-21 antibody; "NC" shown in the figure refers to CV1 cells. F and H show the IL21 content in the culture medium and cell lysate after infecting the cells with the oncolytic vaccinia viruses DDvv-mIL21 and DDvv-hIL21 respectively detected by ELISA kits.

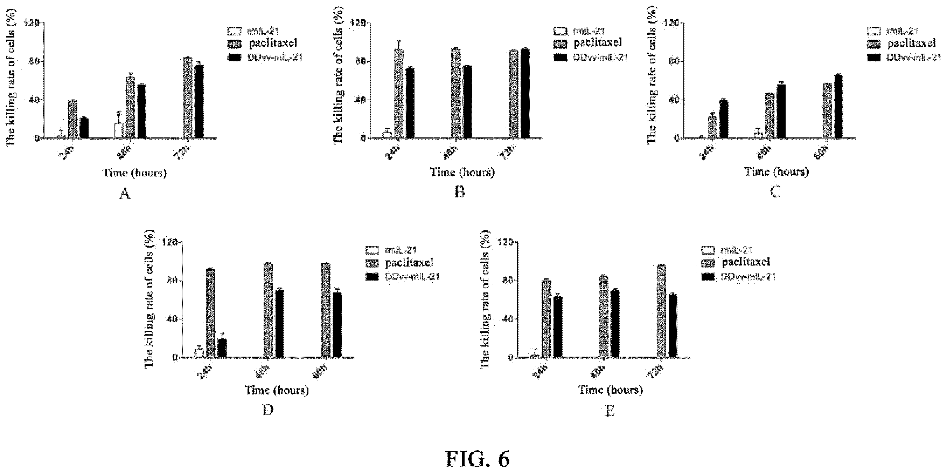

FIG. 6 shows the killing effect of the recombinant vaccinia virus DDvv-mIL21 carrying murine IL-21 fragment on five kinds of mouse-derived cancer cells, wherein A is the experimental result of B16 cell, B is the experimental result of 4T1 cell, and C is the experimental result of LLC cell, D is the experimental result of GL261 cells, E is the experimental result of CT26 cells. Figure 6A-E shows time (hour) on the abscissa and cell killing rate (%) on the ordinate.

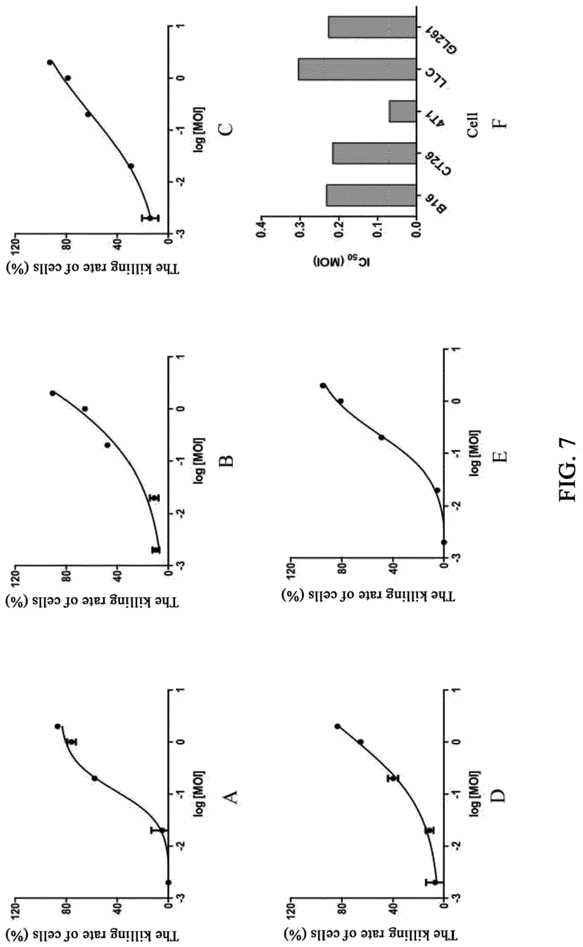

Fig. 7 shows killing effect curve (A-E) and IC₅₀ value (F) of the recombinant vaccinia virus DDvv-mIL21 carrying murine IL-21 fragment on five kinds of mouse-derived cancer cells, wherein A is the experimental result of B16 cells and B is the experimental results of CT26 cells, C is the experimental result of 4T1 cells, D is the experimental result of LLC cells, and E is the experimental result of GL261 cells. Figures 7A-E show the log values of MOI on the abscissa and cell killing rate (%) on the ordinate. Figure 7F shows the cell groups on the abscissa and IC₅₀ value (MOI) on the ordinate.

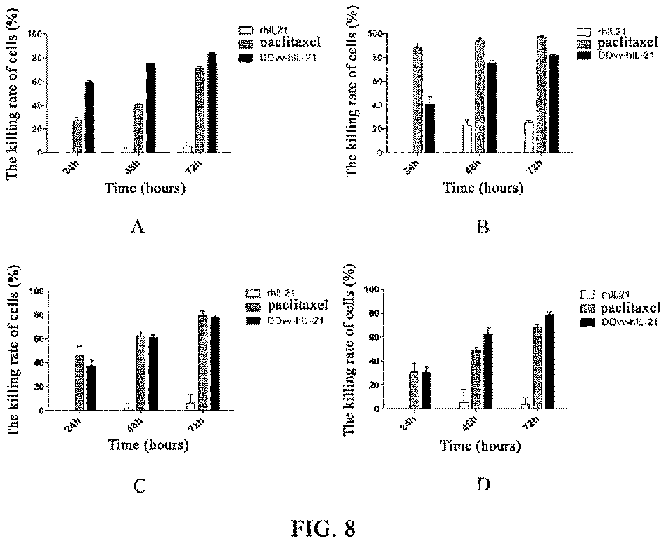

FIG. 8 shows killing effect of the recombinant vaccinia virus DDvv-hIL21 carrying human IL-21 fragments on 4 kinds of human cancer cells, wherein A is the experimental result of A549 cells, B is the experimental result of Hela cells, and C is the experimental result of SKOV3 cells, D is the experimental result of U251 cells. Figures 8A-D show time (hours) on the abscissa and cell killing rate (%) on the ordinate.

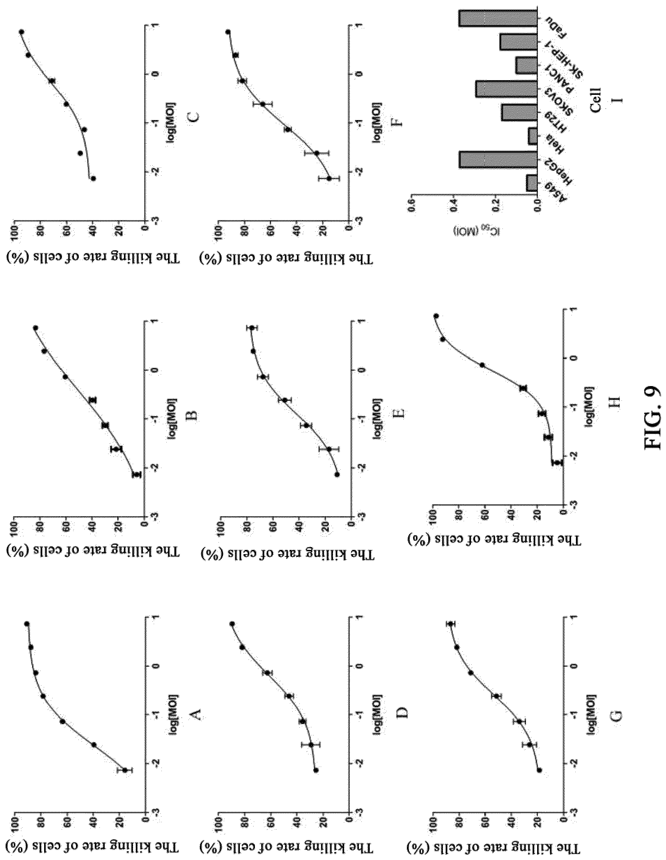

Figure 9 shows killing effect curve (A-H) and IC₅₀ value (I) of the recombinant vaccinia virus DDvv-hIL21 carrying human IL-21 fragment on 8 kinds of human cancer cells, wherein A is the experimental result of A549 cells, B is the experimental results of HepG2 cells, C is the experimental result of Hela cells, D is the experimental result of HT29 cells, E is the experimental result of SKOV3 cells, F is the experimental result of PANC1 cells, G is the experimental result of SK-HEP-1 cells, and H is the experimental result of FaDu cells. Figures 9A-H show log values of MOI on the abscissa and cell killing rate (%) on the ordinate. Figure 9I shows cell groups on the abscissa and IC₅₀ value (MOI) on the ordinate.

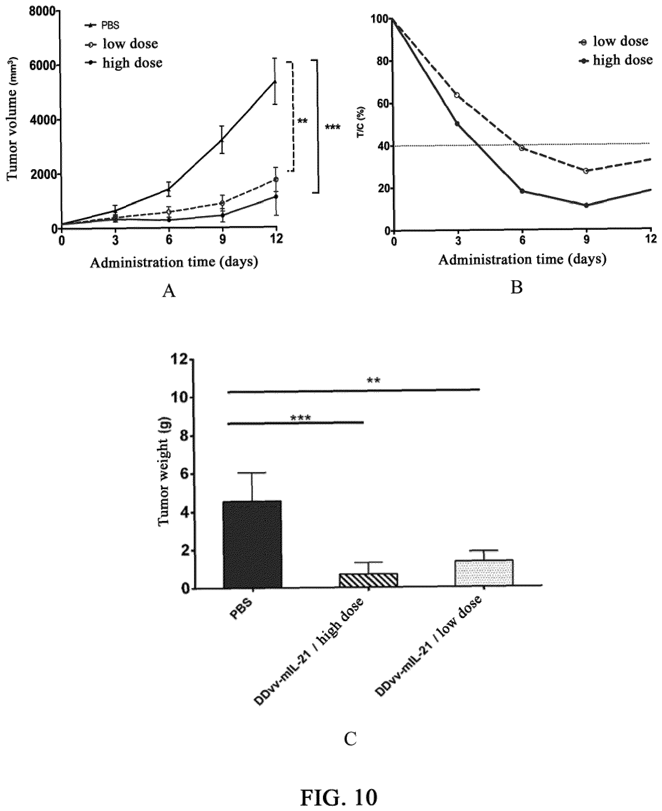

Figure 10 shows antitumor effect of the recombinant vaccinia virus DDvv-mIL21 carrying murine IL-21 fragment on B16 tumor-bearing mice. FIG. 10A shows a curve of tumor volume with time, the abscissa is the administration time (days), and the ordinate is the tumor volume (mm³). FIG. 10B shows the curve of T/C over time, with the abscissa being the time of administration (days) and the ordinate being T/C (%). FIG. 10C shows the comparison results of tumor weights in different dose groups, with the abscissa being the groups and the ordinate being the tumor weight (g).

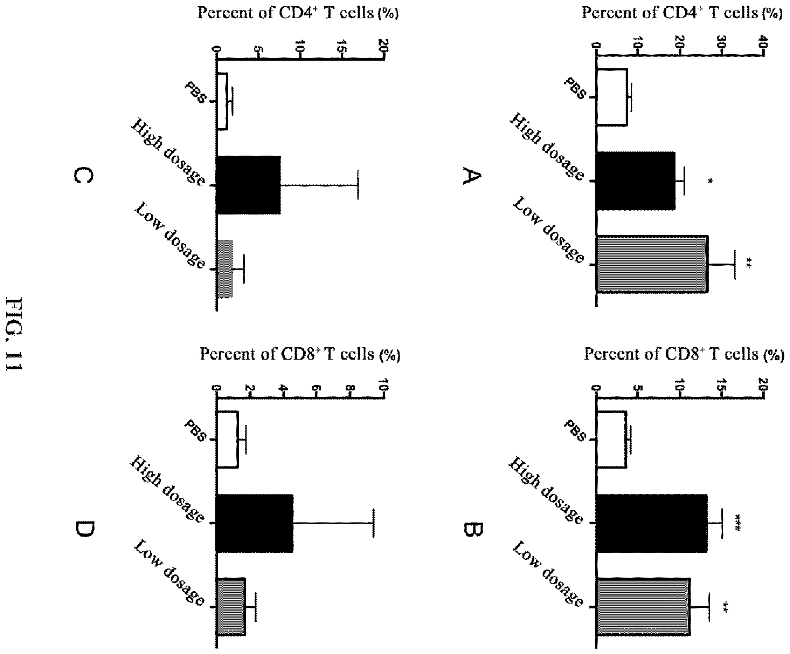

Figure 11 shows the flow cytometric analysis of the immune effect of DDvv-mIL21 on tumor-bearing B16 mice; A and B are flow cytometry analysis of PBMCs extracted from spleen to detect changes in CD4⁺ cells (A) and changes in CD8 ⁺ cells (B); C and D are the flow cytometry analysis of tumor cells extracted from tumor tissue to detect changes in CD4⁺ cells (C) and changes in CD8 + cells (D). In Figures 11A-D, the abscissa is the administration groups, and the ordinate is percentage (%) of CD4 ⁺ T or CD8 ⁺ T cells.

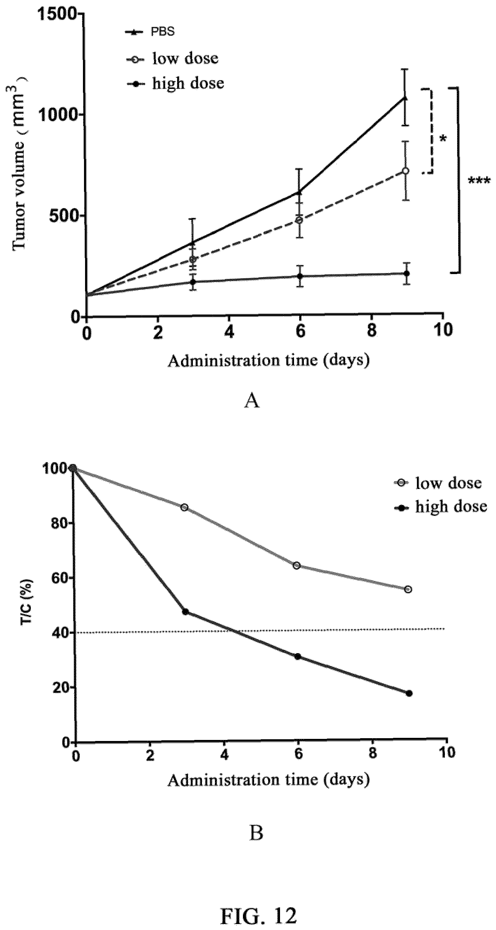

Figure 12 shows tumor-inhibitory effect of the recombinant vaccinia virus DDvv-mIL21 carrying murine IL-21 fragment on LLC tumor-bearing mice. Figure 12A shows curve of tumor volume with time, abscissa is time of administration (days), and ordinate is tumor volume (mm³); Figure 12B shows the curve of T/C with time, wherein abscissa is time of administration (days), and the ordinate is T/C (%).

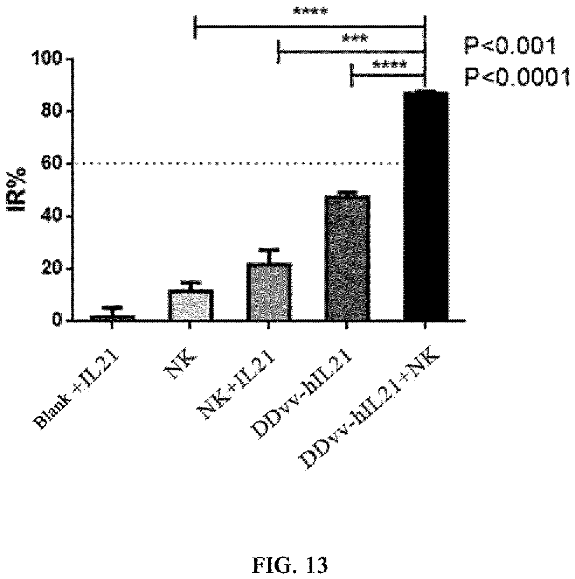

Figure 13 shows killing effect of DDvv-hIL21 vaccinia virus carrying human IL-21 fragment and NK cells on human tumor cell SK-HEP-1 in combination. The abscissa is the groups, and the ordinate is the percentage value of the corresponding inhibition rate.

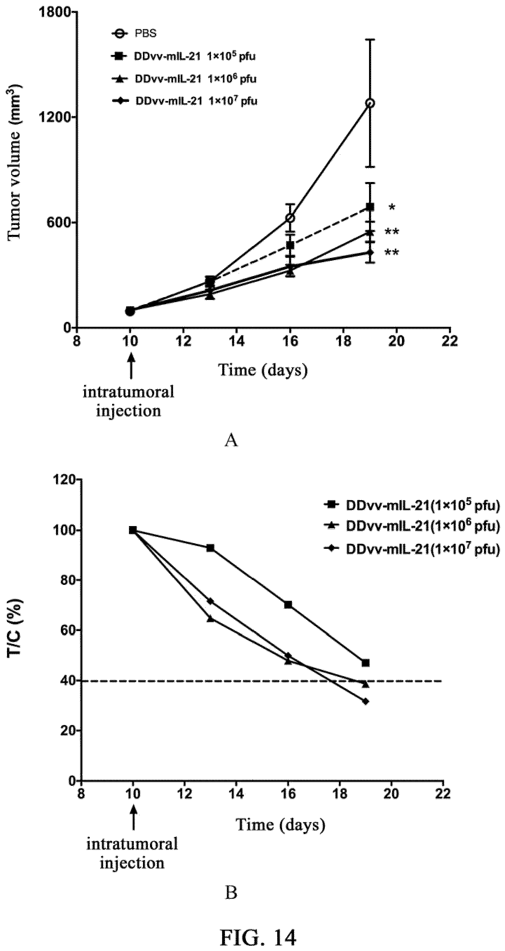

Figure 14 shows antitumor effect of DDvv-mIL21 vaccinia virus carrying murine IL-21 fragment on drug-induced immunocompromised B16 tumor-bearing mice. FIG. 14A shows the change curve of the tumor volume after treatment, wherein the abscissa is the B16 tumor cell inoculation time (days), the arrow on the abscissa indicates the time point of DDvv-mIL21 administration, and the ordinate is the tumor volume (mm³). FIG. 14B shows the change curve of T/C after treatment, the abscissa is B16 tumor cell inoculation time (days), and the ordinate is T/C (%).

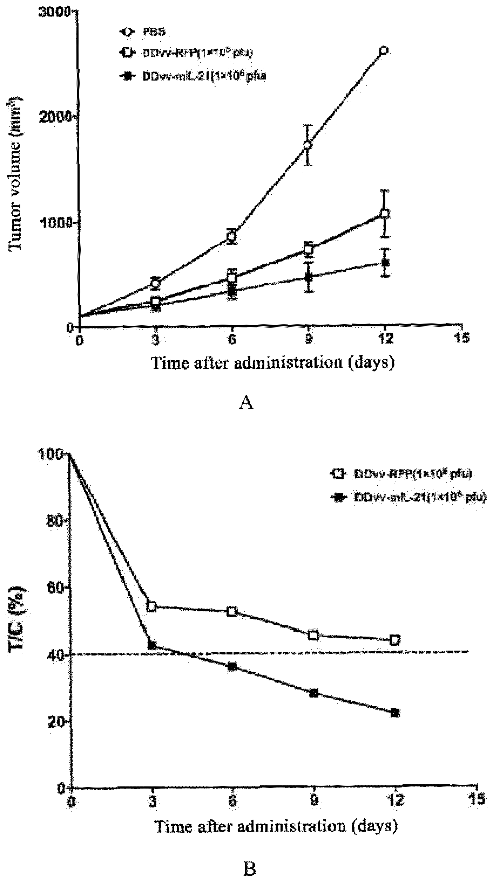

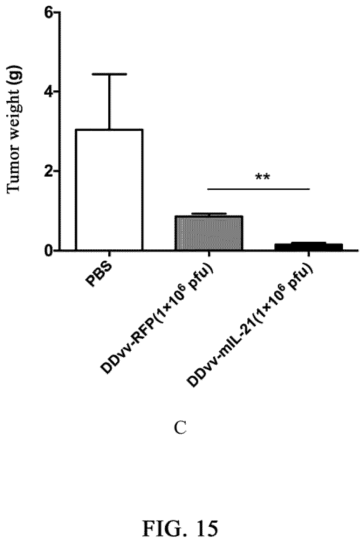

FIG. 15 shows a comparison of the antitumor effect of the DDvv-mIL21 vaccinia virus carrying the mouse IL-21 fragment and the control vaccinia virus on drug-induced immunocompromised B16 tumor-bearing mice. FIG. 15A shows a change curve of the tumor volume after treatment, wherein the abscissa is the time after administration (days), and the ordinate is the tumor volume (mm³); FIG. 15B shows the change curve of T/C after treatment, wherein the abscissa is time after administration (days), the ordinate is T/C (%); FIG. 15C shows the tumor weight after treatment, the abscissa is the groups, and the ordinate is the tumor weight (g).

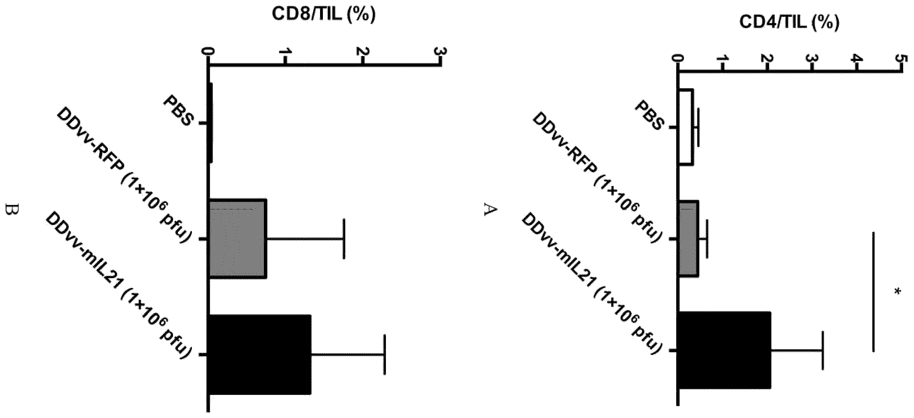

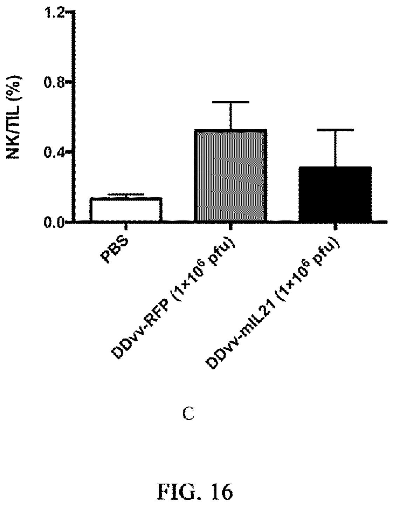

FIG. 16 shows a flow cytometric comparison of the immune effect of a DDvv-mIL21 vaccinia virus carrying a mouse IL-21 fragment and a control vaccinia virus on drug-induced immunocompromised B16 tumor-bearing mice. Figures 16A-C show the changes of CD4⁺ cells (A), CD8⁺ cells (B), and NK cells (C) detected by flow cytometry. In Figures 16A-C, the abscissa is the administration groups, and the ordinate is the percentage (%) by number of CD4⁺ T cells in tumor tissue relative to total immune cells in tumor tissue (A), CD8⁺ T cells in tumor tissue relative to total immune cells in tumor tissue (B) or NK cells in tumor tissue relative to total immune cells in tumor tissue (%) (C).

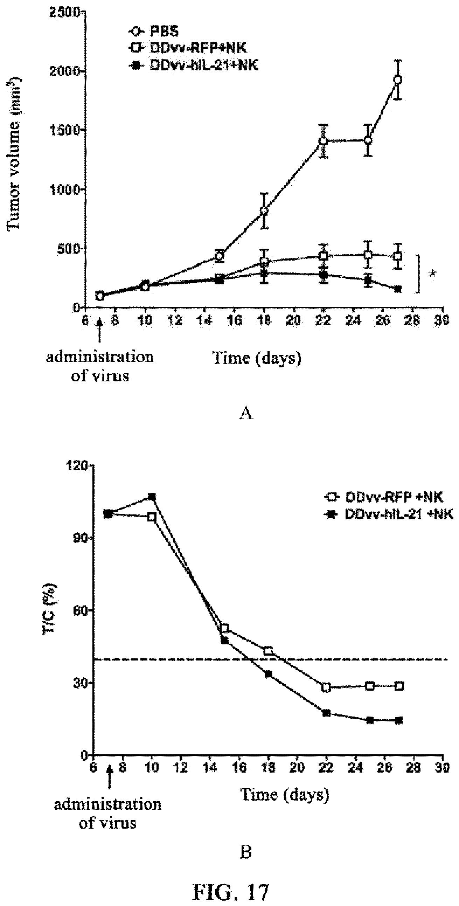

Figure 17 shows a comparison of the antitumor effect of DDvv-hIL21 vaccinia virus carrying human IL-21 fragments combined with human NK cells and control vaccinia virus combined with human NK cells on HCT116 tumor-bearing severely immunodeficiency mice. FIG. 17A shows the change curve of the tumor volume after treatment, the abscissa is the time of inoculation of HCT116 tumor cells (days), the arrow on the abscissa indicates the time point of DDvv-hIL21 administration, and the ordinate is the tumor volume (mm³); FIG. 17B shows the change curve of T/C after treatment, the abscissa is HCT116 tumor cell inoculation time (days), and the ordinate is T/C (%).

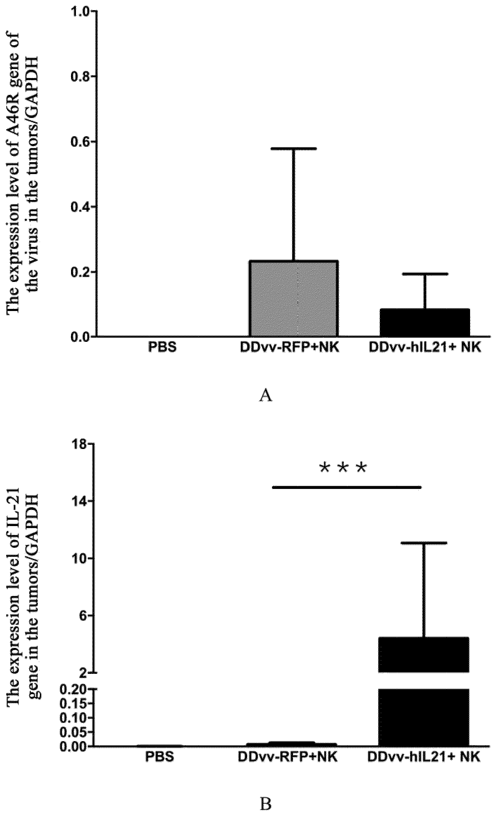

Figures 18A-D show comparison of tumor tissue analysis after treatment of severely immunodeficiency HCT116 tumor-bearing mice with DDvv-hIL21 vaccinia virus carrying human IL-21 fragments combined with human NK cells and control vaccinia virus combined with human NK cell (using quantitative PCR method), wherein the analysis includes the relative expression level of vaccinia virus A46R gene in tumor tissue (A), the relative expression level of IL-21 gene (B), the relative expression level of NKG2D gene in NK cells (C), and the relative expression level of IFN-γ gene (D). In Figs. 18A-D, the abscissa is the administration groups, and the ordinates are the ratio (pg/µg) of the expression level (unit: pg) of each of the target genes in the tumor tissue (A46R gene of the vaccinia virus in the tumor (A), IL-21 gene in the tumor (B), and NKG2D gene of NK cells in the tumor(C) and the IFN--γ gene in the tumor (D)) relative to the expression level (unit: µg) of the GAPDH gene.

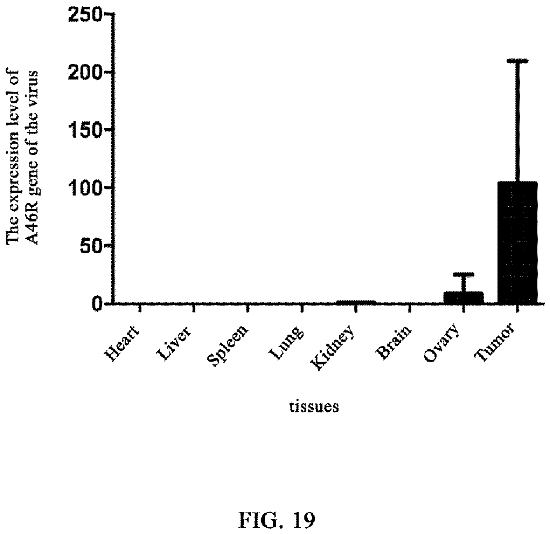

Figure 19 shows the distribution of DDvv-mIL21 vaccinia virus administered intravenously in B16 tumor mice. The abscissa is the type of tissues and organs, and the ordinate is the comparison multiples of the expression amount of vaccinia virus A46R gene in each tissue and organ to the expression amount of vaccinia virus A46R gene in kidney organs, respectively.

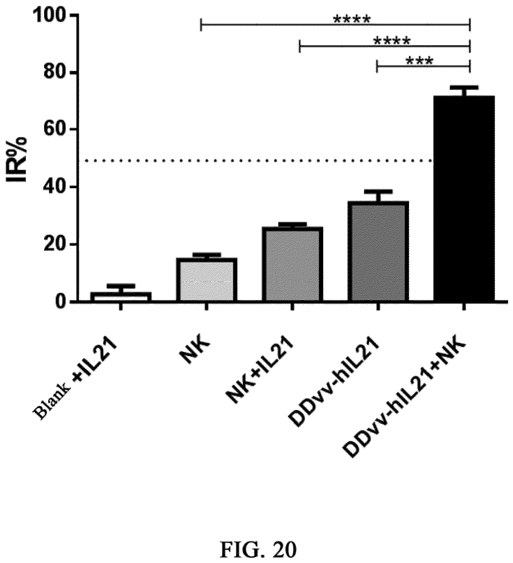

Figure 20 shows killing effect of DDvv-hIL21 vaccinia virus carrying human IL-21 fragment combined with human NK cells on human tumor cell HepG2. The abscissa is the groups, and the ordinate is the percentage value of the corresponding inhibition rate.

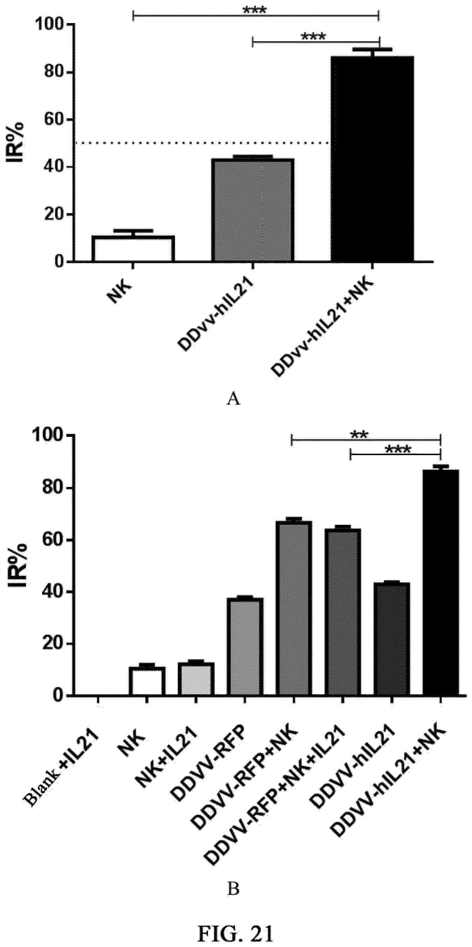

Figure 21 shows a comparison of the killing effect of DDVV-hIL21 vaccinia virus carrying human IL-21 fragments combined with human NK cells and the control vaccinia virus combined with human NK cells on human tumor cell HCT116. FIG. 21A shows a comparison of the NK group, the DDvv-hIL21 group, and the DDvv-hIL21 + NK group;
FIG. 21B shows a comparison of each experimental group. In Figs. 21A and B, the abscissa is the groups, and the ordinate is the percentage value of the corresponding inhibition rate.

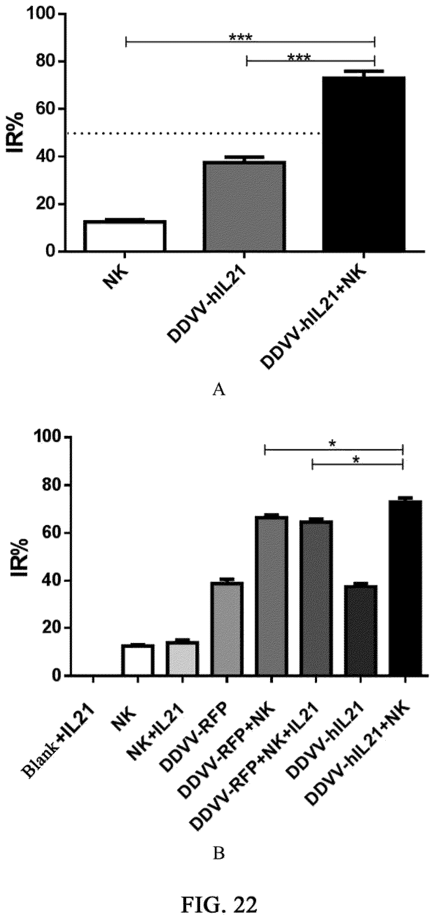

Figure 22 shows a comparison of the killing effect of DDVV-hIL21 vaccinia virus carrying human IL-21 fragments combined with human NK cells and the control vaccinia virus combined with human NK cells on human tumor cells FaDu. FIG. 22A shows a comparison of the NK group, the DDvv-hIL21 group, and the DDvv-hIL21 + NK group; FIG. 22B shows the comparison of each experimental group. In FIGs. 22A and B, the abscissa is the groups, and the ordinate is the percentage value of the corresponding inhibition rate.

In each figure, if shown, * means p <0.05, ** means p <0.01, *** means p <0.001, and **** means p <0.0001 (compared with the control group shown, using One-way ANOVA statistical analysis method).

### DETAILED DESCRIPTION

The present disclosure is further explained with the following detailed description of preferred embodiments with references to the accompanying drawings, which is not to be taken in a limiting sense, and it will be apparent to those skilled in the art that various modifications or improvements can be made accordingly without departing from the spirit of the present disclosure and these are therefore within the scope of the present disclosure.

In the present invention, the words "tumor", "cancer", "tumor cell", and "cancer cell" encompass a meaning commonly recognized in the art.

The human body is a complex system, which is composed of ten major systems such as breathing, circulation, and digestion. These systems coordinate and cooperate to enable various complex life activities in the human body to proceed normally. When tumors occur, the body can exert antitumor effects through a variety of immune effector mechanisms. The body's antitumor mechanisms include cellular immunity and humoral immunity. They are closely related and affect with each other, involving a variety of immune effector molecules and effector cells. It is generally believed that cellular immunity plays a leading role in the anti-tumor process, and humoral immunity plays a synergistic role in some cases. The invention proposes to use the characteristics of oncolytic vaccinia virus to selectively replicate in tumor cells and kill tumor cells, and at the same time to modify the virus with carrying the immune regulatory factor IL-21, so that the recombinant oncolytic vaccinia virus synergistically exerts the function of selective oncolytic and enhancement of the body's anti-tumor immune effect. Based on this concept, the inventors of the present invention discovered through experimental research and theoretical exploration that by rendering both the TK gene and the VGF gene of oncolytic vaccinia virus being functionally deficient and integrating the IL-21 gene in the genome, it is possible to achieve the above-mentioned synergy effect.

Vaccinia virus (VV) is one of the largest and most complex viruses discovered to date, which is the vaccine virus for preventing the variola. The special biological properties of the virus have made it gain more and more attention in the field of tumor immunotherapy/ gene therapy: (1) safety: vaccinia virus is a DNA virus that replicates in the cytoplasm of cells and will not integrate into the host cell genome, reducing mutations induced by foreign genes (see the literature: "Hruby, DE Vaccinia Virus Vectors: New Strategies for Producing Recombinant Vaccines. Clin. Microbiol. Rev. 3, 153-170 (1990)."); (2) High expression efficiency: Vaccinia virus can be cultured to a very high titer (> 10⁹ pfu / ml), and usually 1-3 hours of infection can make more than 90% of infected cells express the gene product of interest (see the literature: "Pfleiderer, M., Falkner, FG & Dorner, F. A novel vaccinia virus expression system allowing construction of recombinants without the need for selection markers, plasmids and bacterial hosts. J. Gen. Virol. 76, 2957-2962 (1995). "); (3) Wide range of infected cells: infecting almost all types of mammalian cells, including dividing and non-dividing cells (see literature: "Hruby, DE Vaccinia Virus Vectors: New Strategies for Producing Recombinant Vaccines. Clin. Microbiol. Rev. 3, 153-170 (1990)."); (4) Large genome capacity: at least 25 kb of foreign genes can be inserted without affecting its genetic stability (see literature: "Hruby, DE Vaccinia Virus Vectors: New Strategies for Producing Recombinant Vaccines. Clin. Microbiol. Rev. 3, 153-170 (1990)."); (5) Stability: insensitive to temperature, good drug-making property, easy to use and transport (see literature: "Hruby, DE Vaccinia Virus Vectors: New Strategies for Producing Recombinant Vaccines. Clin. Microbiol. Rev. 3, 153-170 (1990)."); (6) the expression product has a natural structure: the product expressed can have correct glycosylation and post-translational processing, close to its natural configuration (see the literature: "Hruby, DE Vaccinia Virus Vectors: New Strategies for Producing Recombinant Vaccines. Clin. Microbiol. Rev. 3, 153- 170 (1990). "), Which is essential for viruses to carry immune regulatory genes to stimulate the body's immune response.

The thymidine kinase (TK) gene of vaccinia virus is one of the genes on which the vaccinia virus replication process depends. If the vaccinia virus lacks the TK gene, it is necessary to supply the TK protein through the host cell. However, the TK protein can only be transiently expressed in the normal cell cycle, but the TK protein is consistently high expressed in most tumor cells. Therefore, using the expression characteristics of TK protein in tumor tissues allows the replication of TK-deficient vaccinia virus to be limited to tumor tissues, rather than in normal cells, thereby improving tumor targeting. Since 1982 when the vaccinia virus genetic modification system was started to use, the TK gene region has been an insertion region for foreign genes (see the literature: "Byrd, C. & Hruby, D. Construction of Recombinant Vaccinia Virus. In Vaccinia Virus and Poxvirology: Methods and Protocols 269, 31-40 (2004).").

Second, the intracellular replication and intercellular spread of vaccinia virus is closely related to the activation of epidermal growth factor receptor (EGFR) signaling pathways in host cells. Vaccinia virus infects cells and secretes virus growth factor (VGF), which binds to EGFR on the surface of infected or adjacent uninfected cells and activates the EGFR/Ras signaling pathway which is beneficial for the replication of vaccinia virus and infecting neighboring cells. Therefore, the VGF-deficient vaccinia virus cannot activate EGFR/Ras channels in normal cells, which limits their ability to infect normal cells. However, the EGFR signaling pathway in tumor cells is activated, so the replication and infection of vaccinia viruses lacking the VGF gene in tumor cells are not affected, that is, the specificity of effects on tumor is relatively improved (see the literature: "Autio, K. et al. Safety and biodistribution of a double-deleted oncolytic vaccinia virus encoding CD40 ligand in laboratory Beagles. Mol. Ther. -Oncolytics 1, 1-8 (2014). ").

IL-21 (interleukin-21) is a multi-directional type I cytokine, mainly produced by T cells, which regulates innate and acquired immune responses, and plays an important role in the anti-tumor immune response. The effects of IL-21 on various immune cells and signal transduction have been reported in the literature (see the literature: "Leonard, WJ & Wan, C. IL-21 Signaling in Immunity. F1000Research 5, 1-10 (2016)."). Various types of immune cells mainly include: 1) CD4 ⁺ T cells: promote proliferation and produce cytokines; T_{fh} cells: promote differentiation and improve development center function; Tₕ₁₇ cells: promote differentiation and proliferation; T_{reg} cells: inhibit its production and survival; 2) NKT cells: proliferate and enhance cytotoxicity; 3) CD8⁺ T cells: improve cytotoxicity, proliferation and/or survival, anti-tumor effect; 4) NK cells: promote cell maturation, proliferation, increase cytotoxic effect, and enhance anti-tumor activity; 5) DC cells: inhibit antigen presentation function and induce apoptosis; 6) macrophages: enhance phagocytosis; 7) B cells: promote proliferation and/or apoptosis, promote plasma cell differentiation and immunoglobulin production; 8) In addition, IL-21 can activate a variety of tumor-related signal channels, including JAK / STAT, MARK / PI3K and other signal channels, and regulate the development of tumors. In tumor immunotherapy, activation of cytotoxicity of NK cells and CD8⁺ T cells is the key, and many studies have fully shown that IL-21 plays an important role in this procedure. IL-21 promotes maturation of NK cells to produce IFN-γ and perforin, and induces NK cell-mediated anti-tumor cytotoxicity to target NKG2D ligands on the surface of tumor cells, and increasing the lethality of NK cells via the antibody-dependent cell-mediated cytotoxicity (ADCC) (see literature: "Spolski, R. & Leonard, WJ Interleukin-21: a double-edged sword with therapeutic potential. Nat. Rev. Drug Discov. 13, 379-395 (2014)."). Secondly, IL-21 can induce the proliferation of CD8 ⁺ T cells, induce the generation of memory T cells, and promote the secretion of IFNγ / granzyme to enhance the killing of tumors by CD8⁺ T cells and contribute to the memory immune response to recurrent tumor cells. Importantly, unlike IL-2, IL-21 does not induce the expansion of T_{reg} cells, and further enhances the immune function response of CD8 ⁺ T cells (see literature: "Spolski, R. & Leonard, WJ Interleukin-21: a double-edged sword with therapeutic potential. Nat. Rev. Drug Discov. 13, 379-395 (2014)."). Based on the diverse effects of IL-21 on immune cells, it is shown that IL-21 can "reactivate" a variety of effector cells in the tumor microenvironment. Several clinical studies have used IL-21 alone or in combination with other drugs for tumor treatment.

Therefore, the present invention provides an isolated recombinant oncolytic vaccinia virus, wherein the recombinant oncolytic vaccinia virus is functionally deficient in the TK gene and VGF gene, and the genome of the recombinant oncolytic vaccinia virus is integrated with exogenous IL- 21 gene, and the IL-21 gene is capable of being expressed in tumor cells.

The term "functionally deficient" used in the present invention when referring to the gene of an oncolytic virus means that the oncolytic virus cannot perform the function that the gene should have inherently, that is, the function is lost. This purpose can be achieved by, for example, inserting an exogenous fragment into the gene or knockout of the gene.

Therefore, an exogenous nucleotide sequence can be inserted into the TK gene to make its function deficient. It is also possible to insert an exogenous nucleotide sequence into the VGF gene to make its function deficient, but it is preferable to knock out the VGF gene.

Preferably, the exogenous IL-21 gene is inserted into the TK gene, so that the TK gene can be functionally deficient and the IL-21 gene can be expressed after the infection of tumor cells.

Vaccinia viruses that can be used in the present invention include a Wyeth strain or a WR strain, and an example of the WR strain is VSC20.

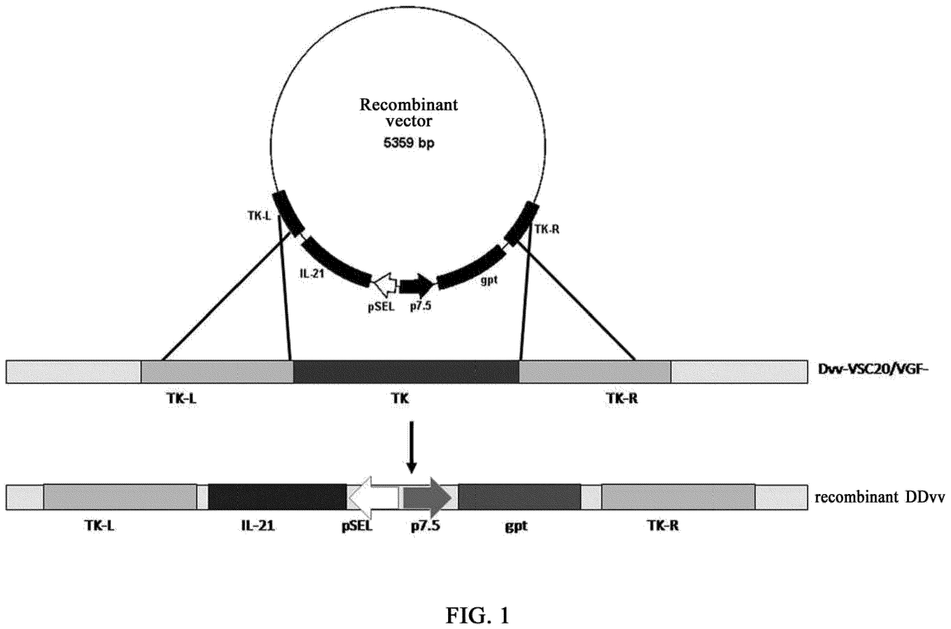

In a preferred embodiment, the recombinant oncolytic vaccinia virus is obtained by genetically modifying the VSC20 vaccinia virus. The VSC20 vaccinia virus is a vaccinia virus lack of VGF gene in which the LacZ gene is inserted at the C11R site. The preparation method thereof can be found in the scientific literature: "McCart, JA, et al. Systemic cancer therapy with a tumor-selective vaccinia virus mutant lacking thymidine kinase and vaccinia growth factor genes. Cancer Res (2001) 61: 8751-8757". The genetic modification includes inserting an exogenous IL-21 gene into the TK gene of the VSC20 vaccinia virus, so that the function of the TK gene is defective. Specifically, a profile of a plasmid for inserting the IL-21 gene into the TK gene of the vaccinia virus VSC20, and a schematic diagram of the insertion through a recombination mechanism are shown in FIG. 1.

The genome of the recombinant oncolytic vaccinia virus may also integrate an exogenous screening gene. The exogenous screening gene includes gpt (guanine phosphoribosyl transferase) gene and/or LacZ gene, but does not include fluorescent protein gene, to avoid the potential safety hazards of fluorescent protein expression in patients.

The genome of the recombinant oncolytic vaccinia virus may not be integrated with an exogenous screening gene either.

In some embodiments, the present invention controls the gpt gene by using the vaccinia virus early / late promoter p7.5 respectively, and controls the exogenous IL-21 gene by using the synthetic vaccinia virus early promoter pSEL, wherein the gpt and IL-21 genes were inserted into the TK gene region of the vaccinia virus VSC20 strain by using *in vitro* intracellular recombination technology to construct the oncolytic virus. The two promoters respectively activated the expression of the respective regulated genes in a back-to-back manner.

The present invention further finds that only when the exogenous IL-21 gene is inserted into the TK gene to cause its functional defect, and the function of the VGF gene of the oncolytic vaccinia virus is rendered functionally deficient, can the oncolytic virus selectively replicate in tumor cell, kill tumor cells, and cause subsequent immune responses in the body, and the exogenous IL-21 gene carried by the oncolytic vaccinia virus can directly induce anti-tumor immune effects.

Preferably, the exogenous IL-21 gene is derived from mouse or human.

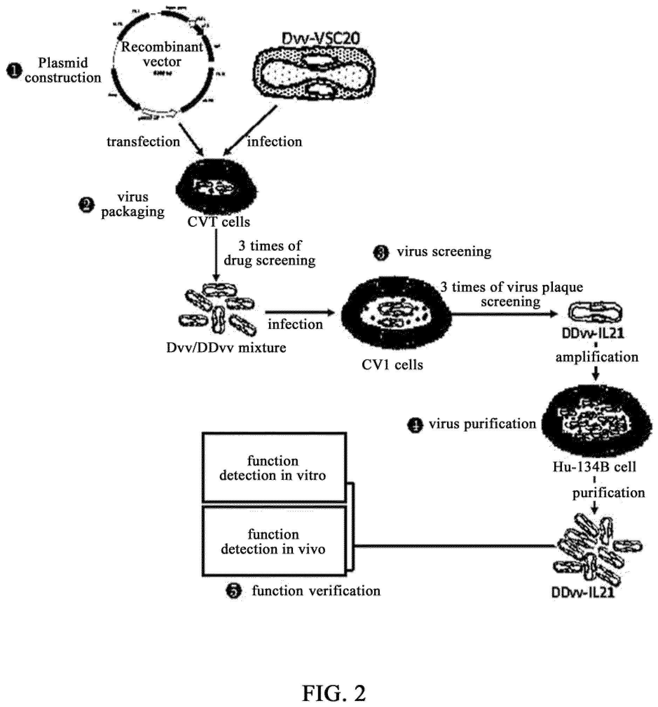

The recombinant oncolytic vaccinia virus of the present invention can be obtained by related known methods in the field of bioengineering, and a specific embodiment is shown in FIG. 2.

The present invention considers the respective characteristics of oncolytic vaccinia virus and IL-21, and skillfully combines them to play their roles, wherein the oncolytic vaccinia virus exerts oncolysis and meanwhile amplifies, via the function of replicating in tumor cells, the expression of IL-21 in tumor cells and then secretion to the outside of the cells to further induce the body's tumor immune effect. The combined effect thereof has a better therapeutic effect.

Based on the recombinant oncolytic vaccinia virus developed by the present invention, the present invention also provides a pharmaceutical composition, wherein the pharmaceutical composition comprises the recombinant oncolytic vaccinia virus according to the present invention as an active ingredient, and a pharmaceutically acceptable excipient.

Preferably, the pharmaceutical composition comprises a therapeutically effective amount of the recombinant oncolytic vaccinia virus. In certain embodiments, the active ingredient of the pharmaceutical composition comprises the recombinant oncolytic vaccinia virus according to the present invention at a dose of 1 × 10⁵ to 5 × 10⁹ pfu/day (eg, 1 × 10⁵ to 3 × 10⁹ pfu/day, 1 × 10⁵ to 1 × 10⁸ pfu/day, etc.).

The recombinant oncolytic vaccinia virus can be administered by the administration methods commonly used in the art, such as intratumoral injection or intravenous administration.

The pharmaceutical composition of the present invention may also contain other active ingredients known in the art, such as interleukin-2 (IL-2), IL-15, IL-18, granulocyte-macrophage colony-stimulating factor (GM-CSF), interferon-γ (IFN-γ), tumor necrosis factor-α (TNF-α), and the like, and the dosage and administration route thereof can be performed in their respective conventional manners. If other active ingredients are included, the recombinant oncolytic vaccinia virus should be present in the pharmaceutical composition independently without being mixed with other active ingredients. For example, the recombinant oncolytic vaccinia virus is individually packaged in a separate container.

Those skilled in the art will understand that the pharmaceutical composition of the present invention may further comprise suitable pharmaceutically acceptable excipients.

In some embodiments, a pharmaceutical composition of the invention comprises one or more pharmaceutically acceptable carriers. Pharmaceutical formulations can be prepared by procedures known in the art. For example, the active ingredients including compounds and the like can be formulated with common excipients, diluents (such as phosphate buffer or saline), tissue-culture medium, and carriers (such as autologous plasma or human serum albumin) and administered as a suspension. Other carriers can include liposomes, micelles, nanocapsules, polymeric nanoparticles, solid lipid particles (see, e.g., E. Koren and V. Torchilin, Life, 63:586-595, 2011). Details on techniques for formulation of the pharmaceutical composition disclosed herein are well described in the scientific and patent literature, see, e.g., the latest edition of Remington's Pharmaceutical Sciences, Maack Publishing Co., Easton PA ("Remington's").

Another aspect of the present invention also provides a vector for preparing the recombinant oncolytic vaccinia virus according to the present invention.

The vector can cause a functional defect in the TK gene and the VGF gene in the vaccinia virus through a recombination mechanism. For example, in a specific embodiment, as shown in FIG. 1, the recombinant vector includes TK homologous fragments TK-L, TK-R, and an expression frame for activating the IL-21 gene and the foreign screening gene gpt. When the vaccinia virus is a WR strain, the sequence of the TK gene is the sequence shown in 80724-81257bp in the vaccinia virus gene numbered NC_006998 in the GenBank of the NCBI (i.e., the National Center for Biotechnology Information, URL: https://www.ncbi.nlm.nih.gov), the sequence of TK-L may be, for example, the sequence fragment shown in 80724-80961 bp, and the sequence of TK-R may be, for example, the sequence fragment shown in 81033-81257bp. The vector can insert the IL-21 gene expression frame and the gpt gene expression frame into the TK gene region of the vaccinia virus through the intracellular recombination mechanism (thus, for example, the sequence fragment shown by the 80962-81032bp in the vaccinia virus gene numbered NC_006998 in GenBank is deleted), so that the recombinant vaccinia virus loses the function of the TK gene. According to another aspect of the present invention, there is provided a host cell comprising the vector of the present invention.

Another aspect of the present invention also provides the use of the recombinant oncolytic vaccinia virus according to the present invention in the preparation of drugs for treating tumors and /or cancers.

The tumor and /or cancer includes, but is not limited to, lung cancer (such as non-small cell lung cancer), melanoma, head and neck cancer, liver cancer, brain cancer, colorectal cancer, bladder cancer, breast cancer, ovarian cancer, uterine cancer, cervical cancer, lymphatic cancer, gastric cancer, esophageal cancer, kidney cancer, prostate cancer, pancreatic cancer, leukemia, bone cancer, testicular cancer, etc.

Another aspect of the present invention also provides a method for treating tumors and/or cancers, comprising administering to a tumor and/or cancer patient the recombinant oncolytic vaccinia virus according to the present invention.

The tumor and /or cancer includes, but is not limited to, lung cancer (such as non-small cell lung cancer), melanoma, head and neck cancer, liver cancer, brain cancer, colorectal cancer, bladder cancer, breast cancer, ovarian cancer, uterine cancer, cervical cancer, lymphatic cancer, gastric cancer, esophageal cancer, kidney cancer, prostate cancer, pancreatic cancer, leukemia, bone cancer, testicular cancer, etc.

In a preferred embodiment of the present invention, the administered dose of the recombinant oncolytic vaccinia virus is a therapeutically effective amount, once a day, for continuous administration for 1-6 days (including continuous administration for 1 day, 2 days, 3 days, 4 days, 5 or 6 days). The therapeutically effective amount is preferably a dose of 1 × 10⁵ to 5 × 10⁹ pfu/day (eg, a dose of 1 × 10⁵ to 3 × 10⁹ pfu/day, a dose of 1 × 10⁵ to 1 × 10⁸ pfu/day, etc.).

If necessary, the recombinant oncolytic vaccinia virus of the present invention can also be used in combination with other drugs, such as interleukin-2 (IL-2), IL-15, IL-18, granulocyte-macrophage colony-stimulating factor (GM-CSF), interferon-γ (IFN-γ), tumor necrosis factor-α (TNF-α), and the like, and the dosage and administration route thereof can be performed in their respective conventional manners .

Based on specific situations and needs, the method for the treatment of tumors and/or cancers according to the present disclosure can be applied to a patient for one time or multiple times.

The recombinant oncolytic vaccinia virus can be administered by the administration methods commonly used in the art, such as intratumoral injection or intravenous administration.

The invention also provides a therapeutic agent, comprising:
(A) a first pharmaceutical composition, wherein the first pharmaceutical composition comprises the recombinant oncolytic vaccinia virus of the present invention in a first pharmaceutically acceptable carrier; and
(B) a second pharmaceutical composition, wherein the second pharmaceutical composition comprises NK cells in a second pharmaceutically acceptable carrier.

In some embodiments, the first and second pharmaceutically acceptable carriers are the same. In other embodiments, the first pharmaceutically acceptable carrier and the second pharmaceutically acceptable carrier are different.

In some cases, the therapeutic agent can also be understood as a combination of drugs.

The mechanisms through which oncolytic viruses kill tumor cells are generally similar. In various embodiments, the oncolytic viruses are administered via intratumoral injection or administered intravenously, and when the oncolytic viruses come into contact with tumor cells, they will infect and enter the tumor cells. Since the oncolytic virus mainly replicates and reproduces in tumor cells with little to no replication in normal cells, large amounts of progeny oncolytic viruses can be produced in the infected tumor cells, leading to lysis and death of the tumor cells. When the tumor cells lyse, large numbers of tumor-associated antigens and the progeny oncolytic viruses may be released, and the antigens can then further activate the immune system *in vivo*, stimulating NK cells and T cells *in vivo* to continue to attack the remaining tumor cells. Meanwhile, the progeny oncolytic viruses can infect the tumor cells which have not been infected yet.

NK cells are immune cells which can kill a broad spectrum of tumor cells, and NK cells can distinguish tumor cells from normal cells. When NK cells come into contact with tumor cells, they can recognize tumor cells as abnormal cells, and will kill the tumor cells through multiple assisting processes such as receptor recognition, target recognition by antibodies (ADCC), as well as releases of granzymes, perforins and interferons capable of killing the tumor cells indirectly. An *in vitro* study has indicated that a healthy NK cell is able to kill up to 27 tumor cells during its life cycle.

NK cells also have anti-virus functions. If normal cells are infected by viruses, the viruses will replicate massively and the infected cells will become aged, showing changes in the composition of protein clusters on the cellular membrane thereof. During this process, NK cells are able to recognize the infected cells sensitively and effectively, and kill these cells with similar approaches they use to kill tumor cells as described above, so as to inhibit replication and proliferation of viruses in the normal cells. Afterwards, with activation of antigens and participation of immune factors like interferons, other types of immune cells will continue to fight against viruses.

In the present disclosure, individual features of the oncolytic virus and NK cells have been taken into account, so they can be combined skillfully. When combined together, the anti-virus mechanism of NK cells is also applicable to tumor cells infected by the oncolytic virus, and this is complementary to the anti-tumor mechanism of NK cells. In addition, the combination therapy allows the tumor cells infected by the oncolytic viruses to become specific targets for NK cells, which can improve their tumor killing effect. The oncolytic viruses not only replicate selectively in cancer cells and kill them from inside, but may also cause the protein receptor clusters on the cellular membrane to change and thus facilitate the recognition of cancer cells by NK cells, so that NK cells can attack the cancer cells from outside. Therefore, the oncolytic virus and NK cells synergistically kill cancer cells, achieving improved efficacy. Further, the recombinant oncolytic vaccinia virus of the present invention also expresses exogenous IL-21, and the expressed exogenous IL-21 can enhance the killing power of NK cells, thereby further enhancing the killing effect of NK cells. The combined use of the recombinant oncolytic vaccinia virus and NK cells described above can produce surprising effects on tumor killing effects.

Preferably, the active ingredient of the first pharmaceutical composition is the recombinant oncolytic vaccinia virus according to the present invention, and the active ingredient of the second pharmaceutical composition is the NK cells.

Preferably, the first pharmaceutical composition and the second pharmaceutical composition are each independently present in the therapeutic agent without being mixed with each other.

In the present invention, the NK cells may be selected from autologous NK cells and allogeneic NK cells; preferably, the NK cells are autologous NK cells obtained from in vitro expansion or allogeneic NK cells obtained from in vitro expansion. Large-scale in vitro expansion culture techniques of NK cells are known and have basically matured (see, for example, the following scientific literature: "Somanchi SS, Lee DA. Ex Vivo Expansion of Human NK Cells Using K562 Engineered to Express Membrane Bound IL21 Methods Mol Biol. 2016; 1441: 175-93. "Or" Phan MT, Lee SH, Kim SK, Cho D. Expansion of NK Cells Using Genetically Engineered K562 Feeder Cells. Methods Mol Biol. 2016; 1441: 167-74 . "). Clinical data confirmed that autologous NK cells, semi-allogeneic NK cells (belonging to allogeneic NK cells), and NK cells prepared from cord blood have no toxic side effects, no long-term dependence, and are safe and effective upon recirculating to the human body.

The purity range of NK cells that can be used for treatment can be: the purity of autologous NK cells may be 85% or more, the purity of allogeneic NK cells may be 90% or more; the impurity cells may be NK-T and/or γδ T cells. Preferably, the NK cell activity (survival rate) is 90% or more, and the NK cell killing activity is 80% or more.

In the combined treatment scheme of the present invention, the present invention further explores and optimizes the respective administration doses of oncolytic virus and NK cells which is essential. Preferably, the first pharmaceutical composition comprises the recombinant oncolytic vaccinia virus at a dose of 1 × 10⁵-5 × 10⁹ pfu/day (eg, the recombinant oncolytic virus at a dose of 1 × 10⁵-3 × 10⁹ pfu/day, the recombinant oncolytic vaccinia virus at a dose of 1 × 10⁵-1 × 10⁸ pfu/day, etc.), and the second pharmaceutical composition contains the NK Cells at a dose of 1 × 10⁷-1 × 10¹⁰ cells/day (preferably, the second pharmaceutical composition comprises the NK cells at a dose of 1 × 10⁸ to 5 × 10⁹ cells/day; also preferably, the second pharmaceutical composition comprises the NK cells at a dose of 1 × 10⁹ to 4 × 10⁹ cells/day; more preferably, the second pharmaceutical composition comprises the NK cells at a dose of 1 × 10⁹ to 3 × 10⁹ cells/day). Preferably, the active ingredient of the therapeutic agent is composed of the recombinant oncolytic vaccinia virus at a dose of 1 × 10⁵ to 5 × 10⁹ pfu/day (eg, the recombinant oncolytic vaccinia virus at a dose of 1 × 10⁵ to 3 × 10⁹ pfu/day, the recombinant oncolytic vaccinia virus at a dose of 1 × 10⁵ to 1 × 10⁸ pfu/day, etc.) and the NK cells at a dose of 1 × 10⁷ to 1 × 10¹⁰ cells/day (eg, 1 × 10⁸ to 5 × 10⁹ cells /day, 1 × 10⁹ to 4 × 10⁹ cells /day, 1 × 10⁹ to 3 × 10⁹ cells /day, etc.).

The recombinant oncolytic vaccinia virus may be administered by the administration methods commonly used in the art, such as intratumoral injection or intravenous administration.

The NK cells can be administered using a method of administration commonly used in the art, for example, intravenously.

Those skilled in the art will appreciate that the therapeutic agents of the present invention may further comprise suitable pharmaceutically acceptable excipients.

The therapeutic agent of the present invention may also contain other active ingredients known in the art, such as interleukin-2 (IL-2), granulocyte-macrophage colony-stimulating factor (GM-CSF), interferon-γ (IFN-γ), tumor necrosis factor-α (TNF-α), etc.

In some embodiments, a therapeutic agent of the invention comprises one or more pharmaceutically acceptable carriers. The pharmaceutical formulations may be prepared by methods known in the art. For example, active ingredients such as compounds can be formulated with common excipients, diluents (such as phosphate buffered saline or saline), tissue culture media, and carriers (such as autologous plasma or human serum albumin) and be administrated as suspensions. Other carriers may include liposomes, micelles, nanocapsules, polymeric nanoparticles, solid lipid particles (see, for example, the document "E. Koren and V. Torchilin, Life, 63: 586-595, 2011"). Specific methods of formulating the therapeutic agents of the present invention can be found in the scientific and patent literatures, for example, see the latest edition of Remington's Pharmaceutical Sciences, Maack Publishing Company, Easton PA ("Remington's").

The therapeutic agent of the present invention can be used for the treatment of a variety of tumors and/or cancers, including but not limited to: lung cancer, melanoma, head and neck cancer, liver cancer, brain cancer, colorectal cancer, bladder cancer, breast cancer, ovarian cancer, uterine cancer, cervical cancer, lymphoma, stomach cancer, esophageal cancer, kidney cancer, prostate cancer, pancreatic cancer, leukemia, bone cancer, testicular cancer, etc.

The application method of the therapeutic agent of the present disclosure is as follows: first, administering the recombinant oncolytic vaccinia virus to a tumor and /or cancer patient, 18-72 hours (e.g., 20-70 hours, 22-48 hours, 24-48 hours, 30-48 hours, *etc.*) after the administration of the recombinant oncolytic vaccinia virus, administering the NK cells to the tumor and/or cancer patient. The phrase "18-72 hours (e.g., 20-70 hours, 22-48 hours, 24-48 hours, 30-48 hours, *etc.*) after the administration of the recombinant oncolytic vaccinia virus, administering the NK cells to the tumor and/or cancer patient" means that the time interval between the first administration of the NK cells and the first administration of the recombinant oncolytic vaccinia virus is in the range of 18-72 hours (e.g., 20-70 hours, 22-48 hours, 24-48 hours, 30-48 hours, *etc.),* or the time interval between the first administration of the NK cells and the most recent administration of the recombinant oncolytic vaccinia virus is in the range of 18-72 hours (e.g., 20-70 hours, 22-48 hours, 24-48 hours, 30-48 hours, *etc.*). Preferably, the time interval between the first administration of the NK cells and the most recent administration of the recombinant oncolytic vaccinia virus is in the range of 18-72 hours (e.g., 20-70 hours, 22-48 hours, 24-48 hours, 30-48 hours, *etc.*). More preferably, the time interval between the first administration of the NK cells and the most recent administration of the recombinant oncolytic vaccinia virus is in the range of 24-48 hours.

In a preferred embodiment of the present invention, the recombinant oncolytic vaccinia virus is administered in a therapeutically effective amount once a day for 1-6 consecutive days; and the NK cell is administered at a dose of 1 × 10⁷ to 1 × 10¹⁰ cells /day (eg, the dose of 1 × 10⁸ to 5 × 10⁹ cells /day, 1 × 10⁹ to 4 × 10⁹ cells /day, 1 × 10⁹ to 3 × 10⁹ cells /day), once a day for 1-6 consecutive days. In another preferred embodiment of the present invention, the administration dose of the recombinant oncolytic vaccinia virus is a therapeutically effective amount, every other day for continuous administration for 2-6 days; and the administration dose of the NK cells is 1 × 10⁷ to 1 × 10¹⁰ cells /day (eg, 1 × 10⁸ to 5 × 10⁹ cells /day, 1 × 10⁹ to 4 × 10⁹ cells /day, 1 × 10⁹ to 3 × 10⁹ cells /day), every other day, continuous administration for 2-6 days. Any one of the above mentioned embodiments or any other alternative embodiment can be adopted according to the present disclosure, as long as the NK cells are to be given to the tumor and/or cancer patient 18 to 72 hours after administration of the recombinant oncolytic vaccinia virus. The recombinant oncolytic vaccinia virus and NK cells may be administered alternatively (for example, administering the recombinant oncolytic vaccinia virus on day 1, administering the NK cells on day 2, administering the recombinant oncolytic vaccinia virus on day 3, and administering the NK cells on day 4, and so on); or may be administered sequentially (for example, administering the recombinant oncolytic vaccinia virus on day 1, administering the recombinant oncolytic vaccinia virus and NK cells in a sequential order on day 2, administering the recombinant oncolytic vaccinia virus and NK cells in a sequential order on day 3, and administering the recombinant oncolytic vaccinia virus and NK cells in a sequential order on day 4, and so on); or may be administered using other dosage regimens (for example, first, administering the recombinant oncolytic vaccinia virus once daily for consecutive 1 to 6 days, and after an interval of 18 to 72 hours, administering the NK cells once daily for consecutive 1 to 6 days). Preferably, the recombinant oncolytic vaccinia virus is administered first, and the NK cells are administered 18 to 72 hours after completion of administrating all the doses of recombinant oncolytic vaccinia virus. In a preferred embodiment of the present disclosure, first, the tumor and/or cancer patient is given the recombinant oncolytic vaccinia virus, and the recombinant oncolytic vaccinia virus is given once only at a therapeutically effective dose; 18 to 72 hours after administration of the recombinant oncolytic vaccinia virus, the tumor and/or cancer patient is administered the NK cells, and the NK cells are administered once only at a dose level ranging from 1×10⁷ to 1×10¹⁰ cells/day (e.g., 1×10⁸ to 5×10⁹ cells/day, 1×10⁹ to 4×10⁹ cells/day, or 1×10⁹ to 3×10⁹ cells/day). The therapeutically effective amount of the recombinant oncolytic vaccinia virus is preferably a dose of 1 × 10⁵ to 5 × 10⁹ pfu /day (eg, a dose of 1 × 10⁵ to 3 × 10⁹ pfu /day, a dose of 1 × 10⁵ to 1 × 10⁸ pfu /day).

The invention also provides the use of the therapeutic agent of the invention in the preparation of drugs for treating tumors and/or cancers.

The tumor and/or cancer includes, but is not limited to, lung cancer (such as non-small cell lung cancer), melanoma, head and neck cancer, liver cancer, brain cancer, colorectal cancer, bladder cancer, breast cancer, ovarian cancer, uterine cancer, cervical cancer, lymphatic cancer, gastric cancer, esophageal cancer, kidney cancer, prostate cancer, pancreatic cancer, leukemia, bone cancer, testicular cancer, etc.

According to another aspect of the present invention, there is also provided a kit of combinational drugs with synergistic effect for treatment of tumors and/or cancers, including a first container containing the recombinant oncolytic vaccinia virus and a second container containing the NK cells according to the present disclosure, wherein the first container is separate from the second container. The kit further comprises instructions specifying timing and routes of administration. Preferably, the kit consists of independent containers respectively containing the recombinant oncolytic vaccinia virus and the NK cells according to the present disclosure, and an instruction sheet specifying timing and routes of administration.

The tumor and/or cancer includes, but is not limited to, lung cancer (such as non-small cell lung cancer), melanoma, head and neck cancer, liver cancer, brain cancer, colorectal cancer, bladder cancer, breast cancer, ovarian cancer, uterine cancer, Cervical cancer, lymphatic cancer, gastric cancer, esophageal cancer, kidney cancer, prostate cancer, pancreatic cancer, leukemia, bone cancer, testicular cancer, etc.

Preferably, the first container containing the recombinant oncolytic vaccinia virus of the present invention contains a therapeutically effective amount of the recombinant oncolytic vaccinia virus, and the second container containing the NK cells contains the NK cells sufficient to provide a dose of 1 × 10⁷-1 × 10¹⁰ cells /day (for example, the NK cells at a dose of 1 × 10⁸ to 5 × 10⁹ cells /day, the NK cells at a dose of 1 × 10⁹ to 4 × 10⁹ cells /day, 1 X 10⁹ to 3 x 10⁹ cells /day of the NK cells, etc.). The therapeutically effective amount of the recombinant oncolytic vaccinia virus is preferably a dose of 1 × 10⁵ to 5 × 10⁹ pfu /day (eg, a dose of 1 × 10⁵ to 3 × 10⁹ pfu /day, a dose of 1 × 10⁵ to 1 × 10⁸ pfu /day).

Preferably, the first container containing the recombinant oncolytic vaccinia virus contains the recombinant oncolytic vaccinia virus at a dose of 1 × 10⁵-1 × 10⁸ pfu /day, and the second container containing the NK cells contains the NK cells at a dose of 1 × 10⁹ to 3 × 10⁹ cells /day.

In the present invention, the NK cells may be selected from autologous NK cells and allogeneic NK cells; preferably, the NK cells are autologous NK cells obtained from in vitro expansion or allogeneic NK cells obtained from in vitro expansion.

The recombinant oncolytic vaccinia viruses can be administered using their respective administration methods commonly used in the art, such as intratumoral injection or intravenous administration.

The NK cells can be administered using the method of administration commonly used in the art, for example, intravenously.

The tumor and/or cancer includes, but is not limited to, lung cancer (such as non-small cell lung cancer), melanoma, head and neck cancer, liver cancer, brain cancer, colorectal cancer, bladder cancer, breast cancer, ovarian cancer, uterine cancer, cervical cancer, lymphatic cancer, gastric cancer, esophageal cancer, kidney cancer, prostate cancer, pancreatic cancer, leukemia, bone cancer, testicular cancer, etc.

Another aspect of the present disclosure also provides a method for treatment of tumors and/or cancers, comprising, in a sequential manner, the following steps:
1) administering the recombinant oncolytic vaccinia viruses according to the present disclosure to a tumor and/or cancer patient;
2) 18-72 hours (e.g., 20-70 hours, 22-48 hours, 24-48 hours, 30-48 hours, *etc.*) after the administration of the recombinant oncolytic vaccinia viruses, administering the NK cells according to the present disclosure to the tumor and/or cancer patient.

The phrase "18-72 hours (e.g., 20-70 hours, 22-48 hours, 24-48 hours, 30-48 hours, *etc.*) after the administration of the recombinant oncolytic vaccinia viruses, administering the NK cells according to the present disclosure to the tumor and/or cancer patient" means that the time interval between the first administration of the NK cells and the first administration of the recombinant oncolytic vaccinia viruses is in the range of 18-72 hours (e.g., 20-70 hours, 22-48 hours, 24-48 hours, 30-48 hours, *etc.),* or the time interval between the first administration of the NK cells and the most recent administration of the recombinant oncolytic vaccinia viruses is in the range of 18-72 hours (e.g., 20-70 hours, 22-48 hours, 24-48 hours, 30-48 hours, *etc.*). Preferably, the time interval between the first administration of the NK cells and the most recent administration of the recombinant oncolytic vaccinia viruses is in the range of 18-72 hours (e.g., 20-70 hours, 22-48 hours, 24-48 hours, 30-48 hours, *etc.*). More preferably, the time interval between the first administration of the NK cells and the most recent administration of the recombinant oncolytic vaccinia viruses is in the range of 24-48 hours.

The tumor and / or cancer includes, but is not limited to, lung cancer (such as non-small cell lung cancer), melanoma, head and neck cancer, liver cancer, brain cancer, colorectal cancer, bladder cancer, breast cancer, ovarian cancer, uterine cancer, cervical cancer, lymphatic cancer, gastric cancer, esophageal cancer, kidney cancer, prostate cancer, pancreatic cancer, leukemia, bone cancer, testicular cancer, etc.

The oncolytic viruses can selectively replicate in tumors or cancer cells and reach a peak after a certain period of time. The inventors of the present invention have found that after a period of replication, the oncolytic virus in tumor cells can promote the killing of tumor cells by NK cells. Therefore, the administration sequence and interval of the recombinant oncolytic vaccinia virus and NK cells proposed by the present invention achieve a double-peak overlap of the peaks of the two effects.

The present invention further explores and optimizes the respective administration doses of the recombinant oncolytic vaccinia virus and NK cells, and the coordination thereof with the above-mentioned administration sequence and interval is crucial, which determines the anti-tumor efficacy of the recombinant oncolytic vaccinia virus, anti-tumor efficacy of NK cells, and the best synergistic killing against tumor cells of the above two.

In a preferred embodiment of the present disclosure, the recombinant oncolytic vaccinia virus is given at a therapeutically effective dose once daily, consecutively for 1 to 6 days; and the NK cells are given at a dose level ranging from 1×10⁷ to 1×10¹⁰ cells/day (e.g., 1×10⁸ to 5×10⁹ cells/day, 1×10⁹ to 4×10⁹ cells/day, or 1×10⁹ to 3×10⁹ cells/day) once daily, consecutively for 1 to 6 days. In another preferred embodiment of the present disclosure, the recombinant oncolytic vaccinia virus is given at a therapeutically effective dose every other day, consecutively for 2 to 6 days; and the NK cells are given at a dose level ranging from 1×10⁷ to 1×10¹⁰ cells/day (e.g., 1×10⁸ to 5×10⁹ cells/day, 1×10⁹ to 4×10⁹ cells/day, or 1×10⁹ to 3×10⁹ cells/day) every other day, consecutively for 2 to 6 days. Any one of the above mentioned embodiments or any other alternative embodiment can be adopted according to the present disclosure, as long as the NK cells are to be given to the tumor and/or cancer patient 18 to 72 hours after administration of the recombinant oncolytic vaccinia virus. The recombinant oncolytic vaccinia virus and NK cells may be administered alternatively (for example, administering the recombinant oncolytic vaccinia virus on day 1, administering the NK cells on day 2, administering the recombinant oncolytic vaccinia virus on day 3, and administering the NK cells on day 4, and so on); or may be administered sequentially (for example, administering the recombinant oncolytic vaccinia virus on day 1, administering the recombinant oncolytic vaccinia virus and NK cells in a sequential order on day 2, administering the recombinant oncolytic vaccinia virus and NK cells in a sequential order on day 3, and administering the recombinant oncolytic vaccinia virus and NK cells in a sequential order on day 4, and so on); or may be administered using other dosage regimens (for example, first, administering the recombinant oncolytic vaccinia virus once daily for consecutive 1 to 6 days, and after an interval of 18 to 72 hours, administering the NK cells once daily for consecutive 1 to 6 days). Preferably, the recombinant oncolytic vaccinia virus is administered first, and the NK cells are administered 18 to 72 hours after completion of administrating all the doses of recombinant oncolytic vaccinia virus. In a preferred embodiment of the present disclosure, first, the tumor and/or cancer patient is given the recombinant oncolytic vaccinia virus, and the recombinant oncolytic vaccinia virus is given once only at a therapeutically effective dose; 18 to 72 hours after administration of the recombinant oncolytic vaccinia virus, the tumor and/or cancer patient is given the NK cells, and the NK cells are given once only at a dose level ranging from 1×10⁷ to 1×10¹⁰ cells/day (e.g., 1×10⁸ to 5×10⁹ cells/day, 1×10⁹ to 4×10⁹ cells/day, or 1×10⁹ to 3×10⁹ cells/day). The therapeutically effective amount of the recombinant oncolytic vaccinia virus is preferably 1 × 10⁵ to 5 × 10⁹ pfu / day (eg, a dose of 1 × 10⁵ to 3 × 10⁹ pfu / day, a dose of 1 × 10⁵ to 1 × 10⁸ pfu / day and so on).

Based on specific situations and needs, the method for the treatment of tumors and/or cancers according to the present disclosure can be applied to a patient for one time or multiple times.

In the present invention, the NK cells may be selected from autologous NK cells and allogeneic NK cells; preferably, the NK cells are autologous NK cells obtained from in vitro expansion or allogeneic NK cells obtained from in vitro expansion.

The tumor and / or cancer include lung cancer, melanoma, head and neck cancer, liver cancer, brain cancer, colorectal cancer, bladder cancer, breast cancer, ovarian cancer, uterine cancer, cervical cancer, lymphoma, gastric cancer, esophageal cancer, kidney cancer, prostate cancer, pancreatic cancer, leukemia, bone cancer, testicular cancer, etc.

The recombinant oncolytic vaccinia virus can be administered by the administration methods commonly used in the art, such as intratumoral injection or intravenous administration.

The NK cells can be administered using a method of administration commonly used in the art, for example, intravenously.

Hereinafter, the present disclosure will be further explained or described by way of examples, but these examples are not intended to limit the scope of protection of the present disclosure.

### Examples

Unless otherwise specified, the experimental methods used in the following examples are performed using routine experimental procedures, operations, materials, and conditions in the field of biological engineering.

Unless otherwise specified, all the percentage concentrations (%) of the respective agents indicate percentage by volume (%(v/v)).

The materials used in the following examples are as follows:
1. CV1 cells and Hu-143B cells are derived from China Center for Type Culture Collection of Wuhan University.
2. Tumor cells
   Except for FaDu human head and neck cancer cells originated from Hangzhou First People's Hospital, the remaining tumor cells are originated from China Center for Type Culture Collection and ATCC. The cells were cultured in normal environment of McCoy's 5A + 10% FBS and MEM + 10% FBS. McCoy's 5A and MEM were purchased from GIBCO. Fetal bovine serum FBS was purchased from SIGMA.
3. NK cells
   The sources of NK cells used in the experiments are as follows:
   The NK cells used in each example were human NK cells cultured and cryopreserved by Hangzhou ConVerd Co., Ltd. The human NK cells were prepared by the following process. As commonly used techniques in the art, a blood collection needle was inserted into an ulnar vein to collect peripheral venous blood of a healthy person for extraction of immune cells PBMCs. Irradiated K562 feeder cells (purchased from Hangzhou Ding Yun Biotech Co., Ltd.) were used to expand NK cells by autologous plasma culture, and the NK cells had a final purity up to 90%, a viability up to 90%, and *in vitro* tumor cell killing rate up to 85%.
4. Control virus
   The oncolytic vaccinia virus ddvv-RFP as the control virus is known, and it belongs to the oncolytic vaccinia virus WR strain (see, for example, "X Song, et al. T-cell Engager-armed Oncolytic Vaccinia Virus Significantly Enhances Antitumor TherapyMolecular Therapy. (2014); 22 1, 102-111"). The oncolytic vaccinia virus ddvv-RFP is functionally deficient in both TK gene and VGF gene, and carries an exogenous red fluorescent protein (RFP) gene. Since the RFP gene only plays a screening/reporting role, the anti-tumor function of the oncolytic vaccinia virus ddvv-RFP is substantially equivalent to the oncolytic vaccinia virus functionally deficient in TK gene and VGF gene. Also, the oncolytic vaccinia virus ddvv-RFP can be obtained by genetic modification of VSC20 vaccinia virus using conventional techniques in the art. VSC20 vaccinia virus is a vaccinia virus lack of VGF gene. For the preparation method of VSC20 vaccinia virus, see "McCart, JA, et al. Systemic cancer therapy with a tumor-selective vaccinia virus mutant lacking thymidine kinase and vaccinia growth factor genes. Cancer Res (2001) 61: 8751-8757". The genetic modification involves the use of an artificial synthetic vaccinia virus early/late promoter pSEL to regulate the exogenous DsRed gene (*i.e.*, the RFP gene), and insertion of the DsRed gene into the TK gene region of the vaccinia virus VSC20 strain using an *in vitro* intracellular recombination technique, thereby constructing the oncolytic vaccinia virus ddvv-RFP.Its production and purification can be carried out according to Preparation Example 2 described below.
5. C57BL / 6 mice were purchased from Beijing vitalriver Experimental Animal Technology Co., Ltd. Severe immunodeficiency NCG mice were obtained from Animal Center of Zhejiang Province.
6. Culture plate
   6-well cell culture plates (2ml culture volume per well), 24-well cell culture plates (500 µl culture volume per well), 96-well cell culture plates (200 µl culture volume per well) are all available from Corning company.
7. Preparation method of drugs for gpt screening: using 0.1M NaOH to prepare 10mg / ml mycophenolic acid (400 ×), 10mg / ml 40 × xanthine (40 ×), 10mg / ml hypoxanthine (670 ×), respectively, and storing under dark at -20°C ; preparing 1 × working solution when used: adding 25 µl mycophenolic acid + 250 µl xanthine + 15 µl hypoxanthine to 10ml DMEM.
8. PBS formula: 8mM Na₂HPO₄, 136mM NaCl, 2mM KH₂PO₄, 2.6mM KCI, pH 7.2-7.4.
9. STE buffer formula: 10mM Tris-Cl, 0.1M NaCl, 1mM EDTA, pH 8.0.
10. The cell counting method used in the following examples is as follows:
   MTT assay: 10µl of MTT solution (5 mg/ml) was added to cells in each well, then the cells were incubated in an incubator at 37°C for 4 hours, the culture medium was drawn and discarded, 150µl of DMSO was added into each well, and then shaken at low speed on a shaker for 10 minutes, allowing the crystal substance to be fully dissolved, and the absorbance value (OD₄₉₀) at 490 nm was measured using a microplate reader. Calculation formula of inhibition rate: Cell proliferation Inhibitory Rate (IR%) = 1- (OD₄₉₀ tested product-OD₄₉₀ blank) / (OD₄₉₀ negative control-OD₄₉₀ blank) × 100%.

Cell counting using Trypan Blue Staining method: the cells were washed with PBS and digested using trypsin, and then the cells were suspended in PBS. To the suspension added Trypan Blue solution to a final concentration of 0.04% (w/v). Then, cell counting was performed under a microscope, during which dead cells were stained blue and living cells appeared transparent without any color. The living cell counts were used as final data.

The abbreviations used in the following examples are explained below:
FBS: Fetal Bovine Serum
PSG: Penicillin-Streptomycin-Glutamine
PBMCs: peripheral blood mononuclear cells
RFP: Red Fluorescent Protein
TILs: tumor infiltrating lymphocytes
DMSO: dimethyl sulfoxide

### Preparation Example 1: Construction of DDvv-IL21 oncolytic vaccinia virus

The following process may be seen in Figure 2.

### (1) Plasmid construction

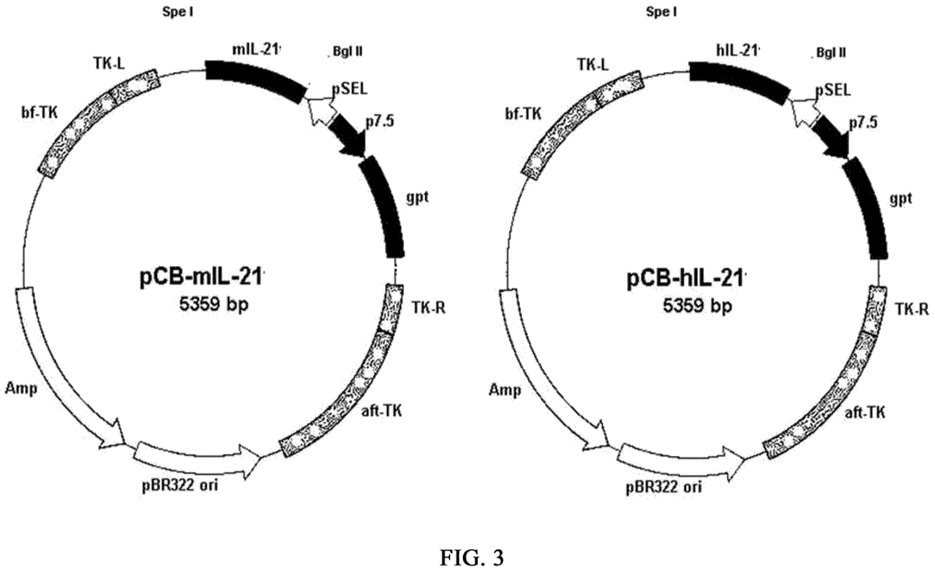

Two strains of oncolytic vaccinia virus were constructed to carry human IL-21 and murine IL-21 genes, respectively, which are labeled DDvv-hIL21 and DDvv-mIL21, respectively.
1) According to NCBI gene library sequences NM_001291041 and NM_021803, a total of 2 mouse IL21 DNA fragments (mIL-21) and human IL21 DNA fragments (hIL-21) were synthesized by gene synthesis methods, specifically:
   mIL-21 contains mouse-derived IL21 fragment (i.e., a 72-560bp fragment in the nucleotide sequence of Genbank number NM_001291041) having a normal sequence, and its sequence (SEQ ID NO.1) is shown below:
      **AGATCT**ATGGAGAGGACCCTTGTCTGTCTGGTAGTCATCTTCTTGGGGACAGT GGCCCATAAATCAAGCCCCCAAGGGCCAGATCGCCTCCTGATTAGACTTCGTCACC TTATTGACATTGTTGAACAGCTGAAAATCTATGAAAATGACTTGGATCCTGAACTTCTA TCAGCTCCACAAGATGTAAAGGGGCACTGTGAGCATGCAGCTTTTGCCTGTTTTCA GAAGGCCAAACTCAAGCCATCAAACCCTGGAAACAATAAGACATTCATCATTGACCT CGTGGCCCAGCTCAGGAGGAGGCTGCCTGCCAGGAGGGGAGGAAAGAAACAGAA GCACATAGCTAAATGCCCTTCCTGTGATTCGTATGAGAAAAGGACACCCAAAGAATT CCTAGAAAGACTAAAATGGCTCCTTCAAAAGGTATGCACCTTAAATGCATTTCTTTCA CTTCCATGTTGTGTCCGGGTACCTCCTGTGCCCAGTGACTCATAGACTAGT; wherein AGATCT is the *Bgl*II recognition sequence; ACTAGT is *Spe*I recognition sequence; and
   hIL-21 contains a human IL21 fragment (i.e., a 47-535bp fragment in the nucleotide sequence of Genbank number NM_021803) having a normal sequence. The sequence (SEQ ID NO. 2) thereof is as follows: 
AGATCTATGAGATCCAGTCCTGGCAACATGGAGAGGATTGTCATCTGTCTGAT
GGTCATCTTCTTGGGGACACTGGTCCACAAATCAAGCTCCCAAGGTCAAGATCGCC
ACATGATTAGAATGCGTCAACTTATAGATATTGTTGATCAGCTGAAAAATTATGTGAAT
GACTTGGTCCCTGAATTTCTGCCAGCTCCAGAAGATGTAGAGACAAACTGTGAGTG
GTCAGCTTTTTCCTGCTTTCAGAAGGCCCAACTAAAGTCAGCAAATACAGGAAACAA
TGAAAGGATAATCAATGTATCAATTAAAAAGCTGAAGAGGAAACCACCTTCCACAAAT
GCAGGGAGAAGACAGAAACACAGACTAACATGCCCTTCATGTGATTCTTATGAGAAA
AAACCACCCAAAGAATTCCTAGAAAGATTCAAATCACTTCTCCAAAAGATGATTCATC
AGCATCTGTCCTCTAGAACACACGGAAGTGAAGATTCCTGAACTAGT;
   wherein AGATCT is the *Bgl*II recognition sequence; ACTAGT is *Spe*I recognition sequence.
   *Bgl*II endonuclease and *Spe*I endonuclease were used to perform cleavage and IL21 gene was inserted into pCB plasmid (it can be obtained according to literature "Yourong FANG et al, Construction of Recombinant Vaccinia Virus Vector with Zeocin and GFP Double Screening labels "journal of international epidemiology and infectious diseases", 2012. 39 (3): 148-152. "). The plasmids pCB-mIL21 and pCB-hIL21 were transformed and extracted using E. coli DH5a (Figure 3).
2) T7 universal primer sequencing was used to detect the plasmids pCB-mIL21 and pCB-hIL21, confirming the sequences were correct.
3) The TIANprep Rapid Mini Plasmid Kit (TIANGEN, DP105-03) was used for medium plasmid DNA extraction.

### (2) Packaging and identification of the recombinant oncolytic vaccinia virus

1) CV1 cells with good growth conditions were selected and spread in a 6-well plate, about 4 × 10⁵ cells / well, so that the cell density reached 80% -90% by the next day.
2) The medium in the 6-well plate was discarded, and 1 ml of serum-free and antibiotic-free DMEM medium containing 9 × 10³ pfu of the Dvv-VSC20 virus (for the source of the virus, see "McCart, JA, et al. Systemic cancer therapy with a tumor-selective vaccinia virus mutant lacking thymidine kinase and vaccinia growth factor genes. Cancer Res (2001) 61: 8751-8757. ") was added to each of the two wells, such that the concentration reached 0.03 pfu / cell, cross-mixed, cultured for 2 hours at 37°C, mixed once every 20 minutes, while setting a control well without adding virus solution.
3) Prepare a plasmid /Lipo2000 (Lipofectamine® 2000 transfection reagent, Life Technologies, 11668-019) mixed solution: Solution A-pipette 5 µg of the plasmid and add it to 300 µl of Opti-MEM I (Gibco, 1802679) until homogenous, incubate for 5 minutes at room temperature; Solution B-pipette 12 µl of Lipo2000, add to 300 µl of Opti-MEM I until homogenous, and incubate at room temperature for 5 minutes; mix the formulated solution A and solution B by mixing gently, incubate at room temperature for 15 minutes to obtain a plasmid / Lipo2000 mixture. Add 300 µl of plasmid / Lipo2000 mixed solution to one of the infected wells, and set up a control well with the transfection plasmid only. Incubate at 37 °C for 4 hours. Replenish 2 ml of DMEM medium containing 10% FBS + 1% PSG to each well.
4) After 24 hours, discard the old medium and replace it with DMEM medium containing 5% FBS + 1% PSG, and continue to culture for about 48 hours. After observing the complete lesion of the cells under a microscope, collect the supernatant and cells, and freeze and thaw repeatedly for 3 times to release the virus.
5) Take out 200 µl of virus solution, use TIANamp Virus DNA/ RNA Kit (TIANGEN, DP315) to extract the virus genome, and use the PCR method to detect whether the inserted IL-21 gene was integrated into the backbone virus; take 50µl virus solution for centrifuge and take the supernatant for the ELISA test (Mouse IL-21 DuoSet ELISA, R & D, DY594-05; Human IL-21 Uncoated ELISA, Invitrogen, 88-8218) to further determine whether the recombinant vaccinia virus expressed IL-21 protein. The remaining virus solution was stored at -80°C, labeled as P0. The primer sequences used for PCR identification are shown in Table 1, and the size of the identification bands obtained by PCR detection of positive viruses is shown in Table 2.

**Table 1. PCR primer sequence**

| **primer** | **sequence** | **Company** |
|---|---|---|
| P1 | ATCGCATTTTCTAACGTGATGGAT (SEQ ID NO.3) | Beijing Qingke Xinye Biotechnology Co., Ltd. |
| P2 | TATCTAACGACACAACATCCATT (SEQ ID NO.4) | |
| P15 | ATGGAATCCGCGTCGACTGAG (SEQ ID NO.5) | |
| P11 | GGCCAGATCGCCTCCTGATTAG (SEQ ID NO.6) | |
| zPl | GGTCCCTGAATTTCTGCCAGC (SEQ ID NO.7) | |

**Table 2. The size of the band of the PCR results**

| **Primer pair** | **The identified virus** | **The size of the band (bp)** |
|---|---|---|
| P1/P2 | VSC20 | 622 |
| P15/P11 | DDvv-mIL21 | 1893 |
| P15/zP1 | DDvv-hIL21 | 1792 |

The PCR results showed that the IL-21 gene sequence was integrated into the TK region of virus, that is, the virus was successfully recombined (Figure 4A-B); the ELISA results showed that the recombinant virus supernatant containing IL21 (shown as "DDvv-mIL-21" in the figure) showed IL-21 protein expression, and the remaining cells (shown as "NC" in the figure), the transfection plasmid (pCB-mIL21), and the VSC20 control showed no IL-21 expression (Figure 4C).

### (3) Screening of the recombinant oncolytic vaccinia virus

1) CV1 cells were cultured in a 60mm petri dish. When reaching 80% full, 1ml of virus dilution was added, which contained 150 µl of P0 virus. After infection for 2 hours, 3ml of screening drug containing 1 × gpt was added for screening. Observing cell lesions and collecting the virus and labeling as P1 virus. Viral genome extraction kit was used to extract virus genes for PCR verification.
2) Repeating the step 1) two times to obtain P3 virus which is verified by PCR method.
3) CV1 cells were cultured in a 10 cm dish until the density reached 80%. Discard the medium, add 3 ml of gradient diluted P3 virus and infect for 2 hours, remove the culture medium, and add 8 ml of 1% (w/v) agarose gel (V_{5% agarose gel}: V_{1 × gptworking solution} = 1: 4) containing 0.8 × gpt screening drug, culture at 37°C for 48 hours, pick a single plaque into 300 µl of sterile PBS, and freeze and thaw repeatedly three times to release the virus. 150 µl of the virus solution was taken from each plaque in a 24-well plate using CV1 cells for small expansion. After about 48 hours when the virus was completely lesioned , the virus was collected and 200 µl of the virus solution was used to extract the genome which was used to verified by PCR method. The remaining virus solution was labeled as P4 and stored at -80 °C.
4) Repeating the step 3) for 2-3 times. The PCR method confirmed that the recombinant virus did not contain the backbone virus VSC20. And, ELISA and Western-blot methods (primary antibody: rabbit anti-mouse IL21, PEpro Tech.INC., # 500-P278; Rabbit anti-IL21 antibody, abcam, ab5978; secondary antibody: Goat anti-Rb, Abcam, ab97051) was used to confirm the expression of IL21 protein.

The results showed that the virus obtained from step 4) above showed no bands in the PCR results of P1/P2 by PCR identification, indicating that the recombinant virus was a pure virus carrying the foreign target gene IL-21, and did not contain its backbone background virus VSC20. (Figures 5A-D). Western blot results also showed that DDvv-IL21 virus can effectively express the IL-21 protein and was of the correct size (Figures 5E, G). ELISA results showed that the IL-21 protein can be detected in the recombinant virus DDvv-IL21 in cell culture supernatants and cell lysate (shown as "Cell" in the figure), which further reflects the characteristic of IL-21 protein as a secreted protein (Figures 5F, H).

### Preparation Example 2: Production and purification of the oncolytic vaccinia virus

### (1) Production and purification

1) Massive amplification of the P5-1 sample of DDvv-mIL21 and the P4-3 sample of DDvv-hIL21. Using 50 of 150mm culture dishes, and when Hu-143B cells grew to about 80%, adding the 50 µL small amplified virus of approximately 2 × 10⁴ PFU to each culture dish to infect cells, and cultured at 37°C and 5% CO₂. After about 2 to 3 days, the cells were observed to become round and beaded under a microscope, and some cells were detached from the petri dish. The culture solution / cells were collected using a cell scraper and stored in a -80 °C refrigerator.
2) Use liquid nitrogen and autoclaved sterile water to repeatedly freeze and thaw the collected virus solution for three times, centrifuge at 2000 rpm for 3 minutes, and collect the supernatant.
3) Centrifuge the collected supernatant at 12,000 rpm for 10 minutes, discard the supernatant, resuspend the pellet in 5 mL of PBS, centrifuge at 12,000 rpm for 10 min, discard the supernatant, and resuspend the pellet in 4 mL of 30% (w/v) sucrose.
4) Preparation of sucrose gradient: 60ml (w / v), 50% (w / v), 40% (w / v) sucrose solution was added to the ultracentrifuge tube orderly from bottom to top, 2mL for each, and the top layer was added with 4mL of resuspended virus solution in 30% (w / v) sucrose.
5) After laying the sucrose gradient, 39,000g ultracentrifugation for 20 minutes, wherein the virus can be observed as a milky white band, distributed in a 50% (w / v) sucrose layer. The virus band was aspirated with a 1mL cut/wide open tip and was placed in a new centrifuge tube (the volume of the aspirated virus band was recorded as V1).
6) Wash the virus with 2 times V1 volume of STE buffer to remove the residual sucrose, centrifuge at 35,000 × g for 1 hour, discard the supernatant, and retain the pellet.
7) Resuspend the pellet with V1 volume of STE, centrifuge at 35,000 × g for 30 minutes, retain the pellet, repeat this operation 2 times, and then wash 3 times with PBS.
8) Finally, resuspend the pellet in pre-chilled PBS, mix and aliquot it in sterile EP tubes, store at -80 °C, and reserve 1 tube at 4 °C for virus titer detection.

### (2) Virus titer detection

1) Use two 6-well Petri dishes, inoculate about 4 × 10⁵ HuTK-143B cells per well, and incubate overnight in a 37 °C, 5% CO₂ incubator such that the next day's cell amount approach 80%-90%.
2) Dilute 200 µl of the virus to be tested with 1800 µl of serum-free DMEM in a 10-fold gradient so that the dilution is in the range of 10⁻⁴-10⁻⁸.
3) Aspirate the cell culture solution in the 6-well plate, add 0.5ml of diluted virus solution, mark the dilution of each well, and set up two blank control wells, infect them at 37 °C for 2 hours, shake the 6-well plate once every 20 minutes to ensure that the wells are evenly wet and to avoid local drying.
4) Add 2 ml of DMEM containing 5% FBS to each well and incubate at 37 °C for 48 hours.
5) Aspirate the culture solution, add 0.5ml of 0.1% crystal violet staining solution to each well, stain at room temperature for 10 minutes, remove the staining solution, wash 3 times with PBS, invert the plate to air dry, and take pictures.
6) Count the number of virus plaques in each well, and the virus titer = the average number of virus plaques / (virus dilution ×volume of the added virus).

For example: If there are 30 virus plaques in a 10⁻⁵ dilution well, the virus titer is 30/ (10⁻⁵ × 0.5) = 6 × 10⁶ pfu/ml.

The titers of DDvv-mIL21 virus and DDvv-hIL21 virus of each batch after amplification / purification were 2 × 10⁸ to 4 × 10⁸ PFU/ml.

### Example 1: Killing effect of DDvv-mIL21 vaccinia virus carrying mouse IL-21 fragment on different mouse tumor cells

Mouse tumor cells were spread on 96-well plates, including B16 cells (mouse melanoma cells), GL261 cells (mouse glioma cells), LLC cells (mouse Lewis lung cancer cells), CT-26 cells (mouse colon cancer cells), 4T-1 cells (mouse breast cancer cells), 5000 cells per well, cultured overnight to get adherence to the wall. The cells were infected with 1MOI DDvv-mIL21 virus prepared by the above method, and tumor cells apoptosis was detected after 24 hours, 48 hours and 60 hours (for LLC cells, GL261 cells), or after 24 hours, 48 hours, and 72 hours (for B16 cells, CT-26 cells, 4T-1 cells) by MTT (n = 3, 2-3 times of repeated experiments). The control groups of this test were: negative control group (no virus infection), recombinant mouse-derived IL-21 protein (rmIL-21, purchased from R & D Systems) and positive control group (1 µM paclitaxel, purchased from Beijing Shuang Lu Pharmaceutical Company), each experimental group and control group were set up with 3 duplicate wells, the cell killing rate was the ratio% of the experimental group to the negative control group. The results showed that killing rate of DDvv-mIL21 virus achieved more than 60% against various tumor cells after 60 hours or 72 hours. Among them, killing rate of DDvv-mIL21 virus against CT-26 and 4T1 cells reached 70% after 24 hours of infection (see Figure 6).

### Example 2: IC₅₀ of the killing effect of DDvv-mIL21 Vaccinia virus carrying murine IL-21 fragment on different murine tumor cells

B16 cells, CT26 cells, 4T1 cells, LLC cells, and GL261 cells were spread in 96-well plates, 5000 cells per well, which were infected using 0.002MOI, 0.02MOI, 0.2MOI, 1MOI, 2MOI of DDvv-mIL21 virus prepared by the above method for 72 hours, and then the cells were tested for cell killing using MTT reagent, and the median lethal dose (IC₅₀ value) of the killing effect of DDvv-mIL21 recombinant vaccinia virus on the different tumor cells was calculated (n = 3, one time experiment). The results showed that the IC₅₀ of the recombinant vaccinia virus against B16 cells, CT26 cells, 4T1 cells, LLC cells, and GL261 cells were 0.232 MOI, 0.216 MOI, 0.07 MOI, 0.304 MOI, and 0.227 MOI, respectively (see Figure 7). It can be seen that the IC₅₀ value of the recombinant vaccinia virus DDvv-mIL21 of the present invention on different tumor cells is low (less than or approximately equal to 0.3 MOI), and it has a good prospect for medicine.

### Example 3: Killing effect of DDvv-hIL21 vaccinia virus carrying human IL-21 fragment on different human tumor cells

A549 (human non-small cell lung cancer cells), SKOV3 (human ovarian adenocarcinoma cells), Hela (human cervical cancer cells), U251 (human neurogliocytoma cells) were spread on a 96-well plate, 5000 cells per well, and upon culturing overnight and adherence to wall the cells were infected with 1 MOI DDvv-hIL21 virus prepared by the above method. After 24 hours, 48 hours, and 72 hours, the MTT assay was used to detect the tumor cell apoptosis (n = 3, 3 times of repeated experiment). The control groups of this test were: negative control group (no virus infection), the recombinant human-derived IL-21 protein (rhIL-21, purchased from R & D Systems) and positive control group (1 µM paclitaxel). Each experimental group and each control group were provided with 3 duplicate wells, and the cell killing rate was the ratio% value of the experimental group to the negative control group. The results showed that the killing effect of the recombinant virus on Hela, A549, SKVO3, U251 cells was time-dependent and strengthened with time (see Figure 8). In addition, the results also showed that DDvv-hIL21 virus had a killing rate of more than 60% on each tumor cell after 48 hours and a killing rate of more than 70% after 72 hours.

### Example 4: IC₅₀ of killing effect of DDvv-hIL21 vaccinia virus carrying human IL-21 fragment on the different human tumor cells

A549, HepG2 (human liver cancer cells), Hela, HT29 (human colorectal cancer cells), SKOV3 (human ovarian adenocarcinoma cells), PANC1 (human pancreatic cancer cells), SKHEP-1 (human liver cancer cells), FaDu (human pharynx squamous carcinoma cells) were spread on a 96-well plate, 5000 cells per well, and the cells were infected with 3-fold diluted MOI gradient of DDvv-hIL21 virus prepared by the above method, so that the MOI concentrations of DDvv-hIL21 virus were: 0.003, 0.01, 0.03, 0.1, 0.3, 1, 3, 10; 48 hours after infection, MTT reagent was used to detect cell killing, and the IC₅₀ value of virus killing on different tumor cells was calculated (n = 3, one time experiment). Each of treatment group and the control group in this study were provided with 3 duplicate wells. The results showed that the IC₅₀ of killing of the recombinant vaccinia virus on various tumor cells were between 0.05 and 0.4 MOI, which were all relatively low (see Figure 9). It can be seen that the recombinant vaccinia virus DDvv-hIL21 of the present invention has a good prospect of medicine.

### Example 5: Antitumor effect of DDvv-mIL21 vaccinia virus carrying mouse IL-21 fragment on B16 tumor mice

Construction of immune healthy tumor-bearing mice: the B16 cells at a logarithmic growth phase were used, and each C57BL/6 mouse (Beijing Vitalriver Experimental Animal Center) was inoculated 200,000 cells subcutaneously on the back of the hind legs to observe tumor growth. When the tumor volume was measured at about 100-200 mm³, the administration was initiated. The mouse was treated by intratumoral administration of 100 µl PBS containing 5 × 10⁶ pfu (low dose), 1 × 10⁷ pfu (high dose) of DDvv-mIL21 prepared by the above method and PBS, respectively, 3 mice per group. The tumor size and body weight of the mouse were measured every 3 days. The results showed that the recombinant virus DDvv-mIL21 could effectively inhibit tumor growth and was dose-dependent (Figure 10A). As of the 9th day after the administration, the T/C (the tumor suppression effectiveness, that is, the percentage ratio of the tumor size in the administration group to the tumor size in the control group, which is effective when the value is less than 40%) of each administration group was less than 40% (Figure 10B). Mice were sacrificed on day 12 and tumors were removed to detect tumor weight, which also showed similar results (Figure 10C).

### Example 6: Antitumor immune activation effect of DDvv-mIL21 vaccinia virus carrying mouse IL-21 fragment on B16 tumor mice

B16 cells at logarithmic growth phase were used. Each C57/BI6 mouse was inoculated 200,000 cells subcutaneously on the back of the hind legs. The tumor growth was observed. When the tumor volume was measured at about 100-200 mm³, the administration was initiated. The mouse was treated by intratumoral administration of 100 µl PBS containing 5 × 10⁶ pfu (low dose), 1 × 10⁷ pfu (high dose) of DDvv-mIL21 prepared by the above method and PBS, respectively, 3 mice per group. Mice were sacrificed after 2 weeks to collect spleen and tumor tissue, PBMCs of spleen and tumor cells in tumor tissue were extracted, and CD8 ⁺ T cells (CD3⁺CD8⁺) (antibodies: anti-mouse CD3, anti-mouse CD8a, eBioscience) and CD4 ⁺ T cells (CD3⁺CD4⁺) (antibody: anti-mouse CD4, eBioscience) were detected using flow cytometry. The results showed that the ratio of CD8⁺ T cells and CD4⁺ T cells in the spleen PBMCs of mice given the recombinant virus DDvv-mIL21 was significantly increased compared to the PBS group (Figures 11A-B). At the same time, the analysis of immune cell infiltration in tumor tissue revealed that the proportion of CD8⁺ T cells and CD4 ⁺ T cells (TILs) in the DDvv-mIL2 high-dose group was increased relative to the PBS group (Figures 11C-D).

### Example 7: Antitumor effect of DDvv-mIL21 vaccinia virus carrying murine IL-21 fragment on LLC tumor mice

LLC cells at logarithmic growth phase were used, and each C57BL/6 mouse was inoculated 1 million cells subcutaneously on the back of the hind legs to observe the tumor growth. When the tumor volume was around 100-200 mm³, the administration was initiated. The mouse was treated by intratumoral administration of 100 µl PBS containing 1 × 10⁶ pfu (low dose), 1 × 10⁷ pfu (high dose) of DDvv-mIL21 prepared by the above method, respectively or 100 µl PBS as control groups, 5 mice per group. The tumor size and body weight of each group were measured every 3 days. The results showed that the recombinant virus DDvv-mIL21 could effectively inhibit the growth of LLC tumors in a dose-dependent manner (Fig. 12A). On day 9 after administration, the T/C of the high-dose administration group was less than 40% (Fig. 12B).

### Example 8: Killing effect of DDvv-hIL21 vaccinia virus carrying human IL-21 fragment combined with NK cells on human tumor cell SK-HEP-1

SK-HEP-1 cells were seeded into a 24-well culture plate, covered with 30%, and incubated at 37 °C and 5% CO₂ for 24 hours in a MEM + 10% FBS environment. MOI = 0.15 of the DDvv-hIL21 virus prepared by the above method was added in a DMEM serum-free environment. After infected for 6 hours, MEM + 10% FBS was added for medium replacement, and cultured at 37 °C and 5% CO₂ for 24 hours. Then fresh MEM + 10% FBS was added for medium replacement and NK cells were added (effector-to-target cell ratio NK: SK-HEP-1 = 5: 1), continued to incubate for 48 hours, the dead cells and debris were washed away. The remaining live SK-HEP-1 cells were counted by trypan blue staining. The experimental group was DDvv-hIL21 + NK group. In the experiment, one group of SK-HEP-1 cells was not added with virus and NK, as the blank group; one group was added with DDvv-hIL21 virus at the corresponding time points but without adding NK, as DDvv-hIL21 virus Group; one group was added with NK at the corresponding time points but was not added with DDvv-hIL21 virus, as the NK group; one group was added with NK at the corresponding time points and simultaneously added with human IL21 recombinant protein at a final concentration of 50ng/ml, but was not added with DDvv-hIL21 virus, as the NK + IL21 group; one group was not added with virus and NK, but human IL21 recombinant protein with a final concentration of 50ng/ml was added at the corresponding time points, as a blank + IL21 group. Experiments for each group were repeated more than three times, and the average value was taken for statistical analysis.

The results are shown in Figure 13 (wherein the abscissa is different groups, and the ordinate is the percentage value of the corresponding inhibition rate). The combination of DDvv-hIL21 virus and NK cells (the oncolytic virus was administered firstly, and NK cells were administered later) showed significant synergistic killing effect on SK-HEP-1, and the synergistic inhibition rate is about 87%. In this experiment, the inhibition rate of DDvv-hIL21 virus alone was approximately 47%, the inhibition rate of NK cells alone was approximately 11%, the inhibition rate of the NK + IL21 group was approximately 22%, and the inhibition rate of the blank + IL21 group was about 1%. The inhibition rate of the blank group was about 0 (not shown in FIG. 13).

The results showed that the combined use of DDvv-hIL21 virus and NK cells (the oncolytic virus was administered firstly and the NK cells were administered later) had a significant synergistic killing effect on SK-HEP-1 cell, and the synergistic inhibition rate was about 87%.

### Example 9: Antitumor effect of DDvv-mIL21 vaccinia virus carrying murine IL-21 fragment on the drug-induced immunocompromised B16 tumor-bearing mice

Construction of the drug-induced immunocompromised tumor-bearing mice: B16 cells at a logarithmic growth phase were used, and each C57BL/ 6 mouse were inoculated 200,000 cells subcutaneously on the back of the hind legs to observe tumor growth. When the tumor volume was measured around 80-150 mm³, intraperitoneal injection of 30 mg/kg cyclosporine every day was initiated until the end of the experiment. On the next day (i.e., 10 days after vaccination), the mouse was treated by intratumoral administration of 100 µl PBS containing 1 × 10⁵ pfu (low dose), 1 × 10⁶ pfu (middle dose), 1 × 10⁷ pfu (high dose) of DDvv-mIL21 prepared by the above method and 100 µl PBS, respectively, 5 mice per group. The tumor size and body weight were measured every 3 days. The results showed that the recombinant virus DDvv-mIL21 could effectively inhibit tumor growth and was dose-dependent (Figure 14A). As of the 9th day after the administration (ie, the 19th day after the inoculation), the T/Cs (effective tumor inhibition rate, that is, the percentage of the tumor size in the administration group to the tumor size in the control group, which indicates that the drug is effective when lower than 40%) of middle dose group and high dose group were less than 40% (Figure 14B).

### Example 10: Comparison of the antitumor effect of DDvv-mIL21 vaccinia virus carrying mouse IL-21 fragment and control vaccinia virus on drug-induced immunocompromised B16 tumor-bearing mice

The B16 cells at a logarithmic growth phase were used. Each C57BL/6 mouse was inoculated with 200,000 cells subcutaneously on the back of the hind legs. The tumor growth was observed. When the tumor volume was measured around 80-150 mm³, intraperitoneal injection of 30 mg / kg cyclosporine every day was initiated until the end of the experiment. On the next day, the mouse was treated by intratumoral administration of 100 µl PBS containing 1 × 10⁶ pfu of DDvv-mIL21 prepared by the above method, 1 × 10⁶ pfu DDvv-RFP and 100µl PBS, respectively, 3 mice per group. The tumor size and body weight of each group were measured every 3 days. The results showed that compared with DDvv-RFP, DDvv-mIL21 can effectively inhibit tumor growth (Figure 15A). From day 6 after administration, the T/Cs of the DDvv-mIL21 group were all less than 40% (Figure 15B). After the experiment, mice were sacrificed and tumor tissues were collected and weighed. The tumor weight in the DDvv-mIL21 group was significantly reduced (Figure 15C). At the same time, the content of TILs (including CD8⁺ T cells (CD3 ⁺ CD8 ⁺) (antibodies: anti-mouse CD3, anti-mouse CD8a, eBioscience) and CD4 ⁺ T cells (CD3⁺CD4⁺) (antibodies: Anti-mouse CD3, anti-mouse CD4, eBioscience)) and NK cells (CD3⁻NK1.1⁺) (antibody: anti-mouse NK1.1, eBioscience) in tumor tissues were detected by flow cytometry. The results showed that the administration of DDvv-RFP and DDvv-mIL21 could significantly improve the TIL content in the tumor tissues. Compared with the DDvv-RFP group, it was showed that CD4⁺T cells in tumor TILs were significantly increased in the DDvv-mIL21 group (Figures 16A-C).

### Example 11: Comparison of antitumor effects of DDvv-hIL21 vaccinia virus carrying human IL-21 combined with human NK cells and the control vaccinia virus combined with human NK cells on severely immunodeficiency mice bearing HCT116 tumors

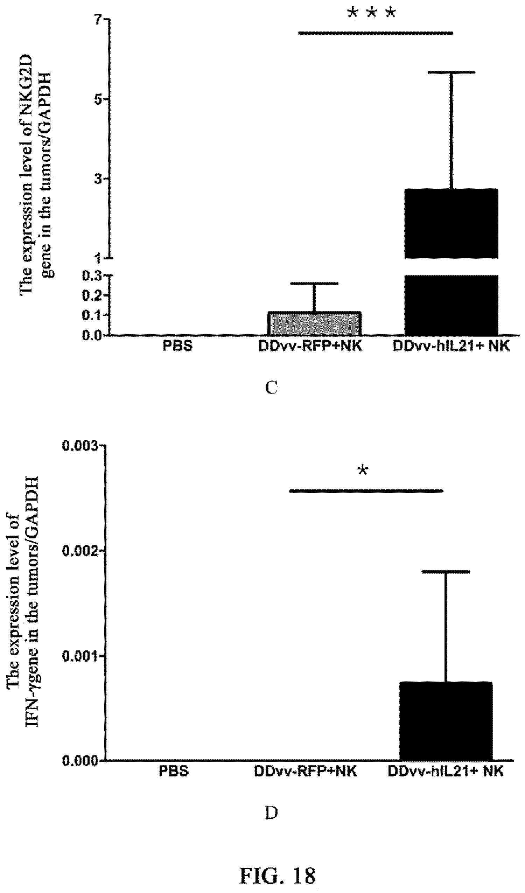

Severe immunodeficiency NCG mice (obtained from Animal Center of Zhejiang Province) were used to inoculate 5 million HCT116 cells subcutaneously on the back of the hind legs of the mice. The tumor growth was observed. When the tumor volume was measured at about 80-150 mm³ (at about 7th day after inoculation), the mouse was treated by intratumoral administration of 100 µl PBS containing 5 × 10⁶ pfu of DDvv-hIL21 viruses, 5 × 10⁶ pfu control vaccinia virus and 100µl PBS, respectively, 5 mice per group. From the next day, each mouse was administered at tail vein with 5 × 10⁷ NK cells / day, and the administration was continued for 3 days, and was stopped for 3 days as a cycle, and a total of 3 cycles of NK cells were administered. Tumor size and body weight were measured every 2-5 days from the start of virus administration. The results showed that both oncolytic vaccinia viruses DDvv-RFP and DDvv-hIL21 can effectively inhibit tumor growth, and DDvv-hIL21 showed better tumor suppressive effect than DDvv-RFP (Figure 17A). Both of the DDvv-RFP and DDvv-hIL21 groups had T/C of less than 40% from 12 days after the administration (Figure 17B). After the experiment, the mice were sacrificed and the tumor tissue was collected. The vaccinia virus in the tumor tissue was detected by quantitative PCR in the expression level of the A46R gene (the primer sequences of the target gene were: 5'-CAGGGAAACGGATGTATA-3' (SEQ ID NO. 8) and 5' -TGTGTTACAGAATCATATAAGG-3'(SEQ ID NO.9)), the expression level of IL-21 gene (the primer sequences of the target gene were: 5'-CCAACTAAAGTCAGCAAATACAGG-3' (SEQ ID NO.10) and 5'-CTTTCTAGGAATTCTTTGGGTGG-3 ' (SEQ ID NO.11)), and the expression level of NKG2D gene in NK cells (the primer sequences of the target gene were: 5'-GGCTTTTATCCACAAGAATCAAGATC-3' (SEQ ID NO.12) and 5'-GTGCACGTCTACCGCAGAGA-3' (SEQ ID NO .13)), the expression level of IFN-γ gene (the primer sequences of the target gene were: 5'-AGTGTGGAGACCATCAAGGAAG-3 '(SEQ ID NO. 14) and 5'-GTATTGCTTTGCGTTGGACAT-3' (SEQ ID NO. 15)), in which house keeping gene GAPDH was used as internal reference (the primer sequences of the target gene were: 5'-GGTCTCCTCTGACTTCAACA-3' (SEQ ID NO.16) and 5'-AGCCAAATTCGTTGTCATAC-3' (SEQ ID NO.17)). The target gene has an expression amount of pg/µg GAPDH. The results showed that the replication of DDvv-hIL21 oncolytic virus in tumors was not significantly different from the control vaccinia virus DDvv-RFP (Figure 18A), and the expression of IL21 further verified the existence of DDvv-hIL21 oncolytic virus in tumor tissues (Figure 18B). Compared with the control group which was administered with DDvv-RFP virus, it was found that the NK content in tumors was significantly increased in the group administered with DDvv-hIL21 oncolytic virus (Figure 18C), and the expression of the immune factor INF-γ was also increased (Figure 18D).

### Example 12: Distribution of DDvv-mIL21 vaccinia virus administered intravenously in B16 tumor mice

The B16 cells in a logarithmic growth phase were used. Each female C57/BI6 mouse was inoculated with 200,000 cells subcutaneously on the back of the hind legs. The tumor growth was observed. When the tumor volume was measured at about 100-200 mm³, the mouse was intravenously administered with 100 µl PBS containing 5 × 10⁹ pfu of DDvv-mIL21 oncolytic virus prepared by the above method, total 4 mice. The mice were sacrificed after 1 week to collect the heart, liver, spleen, lung, kidney, brain, ovary and the tumor tissues. The PCR method was used to detect the distribution of vaccinia virus in various organs. Specifically, the expression amount of A46R gene of the vaccinia virus in each tissue and organ was compared with the expression amount of A46R gene of the vaccinia virus in kidney (the primer sequences of the target gene were: 5'-CAGGGAAACGGATGTATA-3' (SEQ ID NO.8) and 5'-TGTGTTACAGAATCATATAAGG-3' (SEQ ID NO.9)). The results showed that the administered DDvv-mIL21 oncolytic virus was mainly distributed in tumor tissues. The DDvv-mIL21 oncolytic virus was also detected in the ovarian tissue of one mouse among them, and the virus was not detected in other organs (Figure 19).

### Example 13: Killing effect of DDvv-hIL21 vaccinia virus carrying human IL-21 combined with the NK cells on human tumor cell HepG2

HepG2 cells were seeded into a 24-well culture plate, covered with 30%, and incubated in a DMEM + 10% FBS environment at 37 °C and 5% CO₂ for 24 hours. In a DMEM serum-free environment, MOI = 0.027 of DDvv-hIL21 virus prepared by the above method was added to infect cells for 6 hours, and then the solution was changed to DMEM + 10% FBS, and the incubation was continued at 37 °C and 5% CO₂ for 18 hours. Then the liquid was changed to fresh DMEM + 10% FBS, NK cells was added (effector-to-target cell ratio NK: HepG2 = 3: 1), and continued incubation for 48 hours, the dead cells and debris were washed away, and the remaining live HepG2 cells was counted by trypan blue staining. This experimental group is DDvv-hIL21 + NK group. In the experiment, one group of HepG2 cells was not added with virus and NK, as a blank (BK) group. One group was added with DDvv-hIL21 virus at the corresponding time points, but without NK, as a DDvv-hIL21 virus group ; one group was added with NK at the corresponding time points without adding DDvv-hIL21 virus as the NK group; one group was added with NK at the corresponding time points and simultaneously added with human IL21 recombinant protein at a final concentration of 50ng/ml, but without adding DDvv -hIL21 virus, as NK + IL21 group; one group was not added with virus and NK, but added with human IL21 recombinant protein with a final concentration of 50ng /ml at time points corresponding to the above NK addition time points, as a blank + IL21 group; the liquid was changed in above control groups at the corresponding time points. Experiments for each group were repeated more than three times, and the average value was taken for statistical analysis.

The results are shown in Figure 20 (where the abscissa is the different groups, and the ordinate is the percentage value of the corresponding inhibition rate). The combined use of DDvv-hIL21 virus and NK cells (the oncolytic virus was administered firstly, and then NK cells were administered) has significant synergistic killing effect on HepG2, and the synergistic inhibition rate is about 71%. In this experiment, the inhibition rate of DDvv-hIL21 virus alone was about 34%, and the inhibition rate of NK cells alone was about 14%; and the sum of the two is shown by the dotted line in the figure. In addition, the inhibition rate of the NK + IL21 group was about 25%, and the inhibition rate of the blank + IL21 group was about 2%. And the inhibition rate of blank group was about 0 (not shown in FIG. 20).

The results showed that the combined use of DDvv-hIL21 virus and NK cells (the oncolytic virus was administered firstly, and the NK cells were administered later) had a significant synergistic killing effect on HepG2 cells, and the synergistic inhibition rate was about 71%.

### Example 14: Comparison of the killing effect of DDVV-hIL21 vaccinia virus carrying human IL-21 fragments combined with human NK cells and the control vaccinia virus combined with human NK cells on human tumor cell HCT116

It was determined from experiments that the killing dose of DDVV-RFP against HCT116 cells is preferably about MOI = 0.7; the killing dose of NK cells against HCT116 cells is suitably about 5: 1 of the effector to target cell ratio NK: HCT116.

HCT116 cells were seeded into a 24-well culture plate, covered with 30%, and incubated in McCoy's 5A + 10% FBS environment at 37 °C and 5% CO₂ for 24 hours. In the serum-free environment of McCoy's 5A, MOI = 0.7 of the DDVV-hIL21 or DDVV-RFP prepared by the above method was added to infect the cells for 6 hours, and then further incubated in a McCoy's 5A + 10% FBS environment for 18 hours at 37 °C and 5% CO₂. NK cells (effector to target cell ratio NK: HCT116 = 5: 1) were subsequently added (the media was not changed), and the incubation was continued for 48 hours. The dead cells and debris were washed away, and the remaining live HCT116 cells were counted by trypan blue staining. The experimental groups were DDVV-RFP + NK group and DDVV-hIL21 + NK group respectively. In the experiment, one group of HCT116 cells was kept without adding virus and NK as blank group; one group without virus and NK, but the human IL21 recombinant protein was added with a final concentration of 50 ng / ml at time points corresponding to the above NK addition time points, as the blank + IL21 group. One group was only added with DDVV-RFP at the corresponding time points, but without NK, as the DDVV-RFP group; one group was only added with DDVV-hIL21 at the corresponding time points, but without NK, as the DDVV-hIL21 group; one group was added with NK at the corresponding time points, but without virus, as the NK group; one group was added with NK at the corresponding time points, and at the same time, human IL21 recombinant protein was added at a final concentration of 50ng/ml, but no virus was added, as the NK + IL21 group. One group was added with DDVV-RFP and NK at the corresponding time points, and the human IL21 recombinant protein was added with a final concentration of 50ng / ml at the same time points as adding NK, as the DDVV-RFP + NK + IL21 group; the fluid change operations were done for the control groups at the corresponding time points. Experiments for each group were repeated more than three times, and the average value was taken for statistical analysis.

As shown in FIG. 21A (wherein the X-axis is different groups and the Y-axis is the percentage value of the corresponding inhibition rate), the combined use of DDvv-hIL21 virus and NK cells (the oncolytic virus was administered firstly, and NK cells were administered later) has a significant synergistic killing effect on HCT116 cells, and the synergistic inhibition rate is about 86%. In this experiment, the inhibition rate of DDvv-hIL21 virus alone was about 43%, and the inhibition rate of NK cells alone was about 10%; and the sum of the two is shown by a dotted line in the figure. The inhibition rate of the blank group was about 0 (not shown in Figure 21A or B).

In addition, as shown in FIG. 21B (wherein the X-axis is different groups and the Y-axis is the percentage value of the corresponding inhibition rate), the killing effect of the combined use of DDVV-hIL21 and NK cells on HCT116 cells (the inhibition rate was about 86%) is significantly higher than the killing effect of the same dose of DDVV-RFP combined with NK cells (inhibition rate was about 67%), and also significantly higher than the killing effect of the same dose of DDVV-RFP combined with NK cells and human IL21 recombinant protein (inhibition rate was about 64%). The inhibition rate of DDVV-RFP alone was about 37%, the inhibition rate of the blank + IL21 group was not detected, and the inhibition rate of the NK + IL21 group was about 12%. The data of the other groups were shown as above.

The above results indicate that DDVV-hIL21 selectively replicates in HCT116 cells and thus kills HCT116 cells and simultaneously expresses the exogenous IL-2, thereby the expressed exogenous IL-21 enhances the lethality of NK cells and further enhances the killing effect of NK cells on HCT116, such that the combination of DDVV-hIL21 and NK cells exhibits a surprising effect in killing HCT116 cells.

### Example 15: Comparison of the killing effect of DDVV-hIL21 vaccinia virus carrying human IL-21 fragments combined with human NK cells and the control vaccinia virus combined with human NK cells on the human tumor cell FaDu

FaDu cells were seeded into a 24-well culture plate, covered with 30%, and incubated in a MEM + 10% FBS environment at 37 °C and 5% CO₂ for 24 hours. In the serum-free environment of MEM, MOI = 0.2 of DDVV-hIL21 or DDVV-RFP prepared by the method was added to allow for infection for 6 hours, and then further incubated in a MEM + 10% FBS environment at 37 °C and 5% CO₂ for 18 hours. NK cells (effector to target cell ratio NK: FaDu = 5: 1) were subsequently added (the media was not changed), and incubation was continued for 48 hours. Dead cells and debris were washed away. The remaining live FaDu cells were counted by trypan blue staining. The experimental groups were DDVV-RFP + NK group and DDVV-hIL21 + NK group respectively. In the experiment, one group of FaDu cells was not add with virus and NK as a blank group; one group was not add with virus and NK, but a human IL21 recombinant protein was added with a final concentration of 50 ng /ml at a time points corresponding to the above NK addition time points, as the blank + IL21 group. One group was only added DDVV-RFP at the corresponding time points, but without NK, as the DDVV-RFP group; one group was only added with DDVV-hIL21 at the corresponding time points, but without NK, as the DDVV-hIL21 group; one group was added with NK at the corresponding time points, but no virus was added, as the NK group; one group was added with NK at the corresponding time points, and at the same time the human IL21 recombinant protein was added at a final concentration of 50ng / ml, but without virus, as the NK + IL21 group; one group was added with DDVV-RFP and NK at the corresponding time points, and at the same time with the addition of NK, human IL21 recombinant protein at a final concentration of 50 ng/ml was added, as the DDVV-RFP + NK + IL21 group; the corresponding fluid change operations were done for the control groups at the corresponding time points. Experiments for each group were repeated more than three times, and the average value was taken for statistical analysis.

As shown in FIG. 22A (wherein the X-axis is different groups and the Y-axis is the percentage value of the corresponding inhibition rate), the combined use of DDvv-hIL21 virus and NK cells (the oncolytic virus was administered firstly, and NK cells were administered later) has a significant synergistic killing effect on FaDu cells, and the synergistic inhibition rate is about 73%. In this experiment, the inhibition rate of DDvv-hIL21 virus alone was about 37%, and the inhibition rate of NK cells alone was about 13%; and the sum of the two is shown by a dotted line in the figure. The blank group inhibition rate was about 0 (not shown in Figure 22A or B).

In addition, as shown in FIG. 22B (where the X-axis is different groups and the Y-axis is the percentage value of the corresponding inhibition rate), the killing effect of the combined use of DDVV-hIL21 and NK cells on FaDu cells (inhibition rate was about 73%) is significantly higher than the killing effect of DDVV-RFP combined with NK cells at the same dose (inhibition rate was about 66%), and also significantly higher than that of DDVV-RFP combined with NK cells and human IL21 recombinant protein at the same dose (Inhibition rate was about 65%). The inhibition rate of DDVV-RFP alone was about 39%, the inhibition rate of the blank + IL21 group was not detected, and the inhibition rate of the NK + IL21 group was about 14%. The data of the other groups were shown as above.

The above results indicate that DDVV-hIL21 selectively replicates in FaDu cells and thus kills FaDu cells and simultaneously expresses the exogenous IL-21, thereby the expressed exogenous IL-21 increases the lethality of NK cells and thus further enhances the killing effect of NK cells on FaDu, such that the combination of DDVV-hIL21 and NK cells exhibits a surprising effect in killing FaDu cells.

## Claims

1. An isolated recombinant oncolytic vaccinia virus, wherein the recombinant oncolytic vaccinia virus is functionally deficient in the TK gene and the VGF gene, and the genome of the recombinant oncolytic vaccinia virus is integrated with an exogenous IL-21 gene, and wherein the IL-21 gene is capable of being expressed in tumor cells.

2. The recombinant oncolytic vaccinia virus according to claim 1, wherein the TK gene gets functional deficient by being inserted an exogenous nucleotide sequence.

3. The recombinant oncolytic vaccinia virus according to claim 1, wherein the exogenous IL-21 gene is inserted into the TK gene, thereby causing functional defect of the TK gene.

4. The recombinant oncolytic vaccinia virus according to claim 1, wherein the VGF gene gets functional deficient by being knocked out or being inserted an exogenous nucleotide sequence.

5. The recombinant oncolytic vaccinia virus according to claim 1, wherein the recombinant oncolytic vaccinia virus is a Wyeth strain or WR strain.

6. The recombinant oncolytic vaccinia virus according to claim 1, wherein the genome of the recombinant oncolytic vaccinia virus is further integrated with an exogenous screening gene, and the exogenous screening gene includes the gpt gene and/or LacZ gene, but do not include fluorescent protein genes.

7. The recombinant oncolytic vaccinia virus according to claim 1 or 5, wherein the exogenous IL-21 gene is derived from mouse or human.

8. A pharmaceutical composition, wherein the pharmaceutical composition comprises the recombinant oncolytic vaccinia virus according to any one of claims 1-7 as an active ingredient, and a pharmaceutically acceptable excipient.

9. The pharmaceutical composition according to claim 8, wherein the pharmaceutical composition comprises the recombinant oncolytic vaccinia virus at a dose of 1 × 10⁵ - 5 × 10⁹ pfu/day.

10. The pharmaceutical composition according to claim 8, wherein the recombinant oncolytic vaccinia virus is formulated such that it is administered by intratumoral injection or intravenous administration.

11. A vector for preparing the recombinant oncolytic vaccinia virus according to any one of claims 1 to 7, wherein the vector comprises an exogenous IL-21 gene under the control of a promoter.

12. A host cell comprising the vector according to claim 11.

13. Use of the recombinant oncolytic vaccinia virus according to any one of claims 1 to 7 for preparation of drugs for treatment of tumors and/or cancers.

14. The use according to claim 13, wherein the tumors and/or cancers include lung cancer, melanoma, head and neck cancer, liver cancer, brain cancer, colorectal cancer, bladder cancer, breast cancer, ovarian cancer, uterus cancer, cervical cancer, lymphoma, stomach cancer, esophageal cancer, kidney cancer, prostate cancer, pancreatic cancer, leukemia, bone cancer, testicular cancer.

15. A method for treating tumors and/or cancers, comprising administering the recombinant oncolytic vaccinia virus according to any one of claims 1 to 7 to a tumor and /or cancer patient.

16. The method according to claim 15, wherein the recombinant oncolytic vaccinia virus is administered at a dose of 1 × 10⁵ - 5 × 10⁹ pfu/day, once a day, for 1-6 consecutive days.

17. The method according to claim 15, wherein the recombinant oncolytic vaccinia virus is administered by intratumoral injection or intravenous administration.

18. The method according to claim 15, wherein the tumors and /or cancers include lung cancer, melanoma, head and neck cancer, liver cancer, brain cancer, colorectal cancer, bladder cancer, breast cancer, ovarian cancer, uterus cancer, cervical cancer, lymphoma, stomach cancer, esophageal cancer, kidney cancer, prostate cancer, pancreatic cancer, leukemia, bone cancer, testicular cancer.
